# EUROPEAN PATENT SPECIFICATION

(11) **EP 2 138 585 B1**
(45) Date of publication and mention of the grant of the patent: **09.02.2011**
(21) Application number: 09003107.1
(22) Date of filing: 04.03.2009
(51) Int. Cl.: C12P 13/12, C12N 1/20, C12R 1/19

(54) **An L-cysteine producing bacterium and a method for producing L-cysteine**
L-Zystein-produzierendes Bakterium und Verfahren zur Herstellung von L-Zystein
Bactérie productrice de L-cystéine et procédé de production de L-cystéine

(30) Priority: 06.03.2008 JP 2008056371
(43) Date of publication of application: 30.12.2009
(73) Proprietor: Ajinomoto Co., Inc., Tokyo 104-8315 (JP)
(72) Inventor: Nonaka, Gen, Kawasaki-shi Kanagawa 210-8681 (JP); Takumi, Kazuhiro, Kawasaki-shi Kanagawa 210-8681 (JP)
(74) Representative: Strehl Schübel-Hopf & Partner

(56) References cited:
- US-A1- 2005 112 731
- YAMADA S ET AL: "Effect of drug transporter genes on cysteine export and overproduction in Escherichia coli" APPLIED AND ENVIRONMENTAL MICROBIOLOGY JULY 2006 AMERICAN SOCIETY FOR MICROBIOLOGY US, vol. 72, no. 7, July 2006 (2006-07), pages 4735-4742, XP002552828
- AWANO N ET AL: "Effect of cysteine desulfhydrase gene disruption on L-cysteine overproduction in Escherichia coli" APPLIED MICROBIOLOGY AND BIOTECHNOLOGY, SPRINGER VERLAG, BERLIN, DE, vol. 62, no. 2-3, 1 August 2003 (2003-08-01), pages 239-243, XP002331156 ISSN: 0175-7598
- MASARU WADA ET AL: "Metabolic pathways and biotechnological production of l-cysteine" APPLIED MICROBIOLOGY AND BIOTECHNOLOGY, SPRINGER, BERLIN, DE, vol. 73, no. 1, 5 October 2006 (2006-10-05), pages 48-54, XP019441738 ISSN: 1432-0614
- TAKAGI HIROSHI ET AL: "Overproduction of L-cysteine and L-cystine by expression of genes for feedback inhibition-insensitive serine acetyltransferase from Arabidopsis thaliana in Escherichia coli" FEMS MICROBIOLOGY LETTERS, BLACKWELL PUBLISHING, AMSTERDAM, NL, vol. 179, no. 2, 15 October 1999 (1999-10-15), pages 453-459, XP002209321 ISSN: 0378-1097
- DENK D ET AL: "L-CYSTEINE BIOSYNTHESIS IN ESCHERICHIA COLI: NUCLEOTIDE SEQUENCE AND EXPRESSION OF THE SERINE ACETYLTRANSFERASE (CYSE) GENE FROM THE WILD-TYPE AND A CYSTEINE-EXCRETING MUTANT" JOURNAL OF GENERAL MICROBIOLOGY, SOCIETY FOR MICROBIOLOGY, READING, GB, vol. 133, no. 3, 1 March 1987 (1987-03-01), pages 515-525, XP000617605 ISSN: 0022-1287
- DATABASE UniProt [Online] 23 October 2007 (2007-10-23), "SubName: Full=Putative uncharacterized protein;" XP002552804 retrieved from EBI accession no. UNIPROT:A8AK06 Database accession no. A8AK06

## Description

### BACKGROUND OF THE INVENTION

### Field of the Invention

The present invention relates to a method for producing L-cysteine or related substances. More precisely, the present invention relates to a bacterium suitable for the production of L-cysteine or related substances and a method for producing L-cysteine or related substances utilizing such a bacterium. L-cysteine and L-cysteine-related substances are used in the fields of drugs, cosmetics and foods.

### Brief Description of the Related Art

Microorganisms which are able to produce L-cysteine are known. For example, a coryneform bacterium with increased intracellular serine acetyltransferase activity produces L-cysteine (Japanese Patent Laid-open (Kokai) No. 2002-233384). L-cysteine-producing ability can also be increased by incorporating serine acetyltransferase which has been mutated to attenuate feedback inhibition by L-cysteine (Japanese Patent Laid-open No. 11-155571, U.S. Patent Published Application No. 20050112731, U.S. Patent No. 6,218,168).

Furthermore, L-cysteine-producing ability in a microorganism can be enhanced by suppressing the L-cysteine decomposition system. Examples of such microorganisms include coryneform bacteria or *Escherichia* bacteria in which the activity of cystathionine-β-lyase (U.S. Patent Published Application No. 20050112731), tryptophanase (Japanese Patent Laid-open No. 2003-169668), or O-acetylserine sulfhydrylase B (Japanese Patent Laid-open No. 2005-245311) is attenuated or deleted.

Furthermore, it is known that the *ydeD* gene which encodes the YdeD protein participates in secretion of the metabolic products of the cysteine pathway (Dabler et al., Mol. Microbiol., 36, 1101-1112 (2000)). Furthermore, techniques of enhancing L-cysteine-producing ability by increasing expression of the *mar-*locus, *emr*-locus, *acr*locus, *cmr*-locus, *mex*-gene, *bmr*-gene or *qacA-gene,* are also known. These loci and/or genes encode proteins which cause secretion of toxic substances from cells (U.S. Patent No. 5,972,663). *emrAB, emrKY, yojIH, acrEF, bcr* and *cusA* are further examples (Japanese Patent Laid-open No. 2005-287333).

An *Escherichia coli* has been reported which produces L-cysteine and which has increased activity of the positive transcriptional control factor of the cysteine regulon encoded by the *cysB* gene (International Patent Publication WO01/27307).

The *d0191* gene is a novel gene of *Pantoea ananatis* found by the inventor of the present invention. Although genes presumed to be homologue genes of this gene have been found in various bacteria by homology search, functions of all of them are unknown, and relation thereof with L-cysteine production is not known.

### Summary of the Invention

The present invention provides a novel technique for improving bacterial L-cysteine-producing ability, and thereby provide an L-cysteine-producing bacterium, as well as a method for producing L-cysteine, L-cystine, their derivatives, or a mixture of these by using such a bacterium.

A novel gene encoding a protein having cysteine desulfhydrase activity in *Pantoea ananatis* was isolated. L-cysteine-producing ability of a bacterium is enhanced by modifying the bacterium so that the activity of that protein is decreased.

It is an aspect of the present invention to provide a bacterium belonging to the family *Enterobacteriaceae,* which has L-cysteine-producing ability and has been modified to decrease the activity of the protein selected from the group consisting of
(A) a protein comprising the amino acid sequence of SEQ ID NO: 2, and
(B) a protein comprising the amino acid sequence of SEQ ID NO: 2, but which includes substitutions, deletions, insertions or additions of one or several amino acid residues, and having cysteine desulfhydrase activity.

It is a further aspect of the present invention to provide the aforementioned bacterium, wherein the activity of the protein is decreased by decreasing expression of a gene encoding the protein or by disrupting the gene.

It is a further aspect of the present invention to provide the aforementioned bacterium, wherein the gene is selected from the group consisting of:
(a) a DNA comprising the nucleotide sequence of SEQ ID NO: 1, and
(b) a DNA which is able to hybridize with a sequence complementary to the nucleotide sequence of SEQ ID NO: 1 or a probe prepared from the nucleotide sequence under stringent conditions, and encodes a protein having cysteine desulfhydrase activity.

It is a further aspect of the present invention to provide the aforementioned bacterium, in which serine acetyltransferase has been mutated so that feedback inhibition by L-cysteine is attenuated.

It is a further aspect of the present invention to provide the aforementioned bacterium, which is a *Pantoea* bacterium.

It is a further aspect of the present invention to provide the aforementioned bacterium, which is *Pantoea ananatis.*

It is a further aspect of the present invention to provide a method for producing L-cysteine, L-cystine, a derivative thereof or a mixture thereof, which comprises culturing the aforementioned bacterium in a medium and collecting L-cysteine, L-cystine, a derivative thereof or a mixture thereof from the medium.

It is a further aspect of the present invention to provide the aforementioned method, wherein the derivative of L-cysteine is a thiazolidine derivative.

It is a further aspect of the present invention to provide a DNA encoding the protein selected from the group consisting of:
(A) a protein comprising the amino acid sequence of SEQ ID NO: 2, and
(B) a protein comprising the amino acid sequence of SEQ ID NO: 2, but which includes substitutions, deletions, insertions or additions of one or several amino acid residues, and having cysteine desulfhydrase activity.

It is a further aspect of the present invention to provide the aforementioned DNA, which is a DNA selected from the group consisting of:
(a) a DNA comprising the nucleotide sequence of SEQ ID NO: 1, and
(b) a DNA which is able to hybridize with a sequence complementary to the nucleotide sequence of SEQ ID NO: 1 or a probe prepared from the nucleotide sequence under stringent conditions, and encodes a protein having cysteine desulfhydrase activity.

According to the present invention, L-cysteine-producing ability of bacteria can be improved. Furthermore, according to the present invention, L-cysteine, L-cystine, their derivatives, or a combinations thereof can be efficiently produced.

Moreover, the present invention provides a novel gene encoding a protein having cysteine desulfhydrase activity.

### Brief Description of the Drawings

Fig. 1 shows the structure of the helper plasmid RSF-Red-TER.
Fig. 2 shows the construction of the helper plasmid RSF-Red-TER.
Fig. 3 shows the scheme of deletion of the *d0191* gene.
Fig. 4 shows the construction of the pMIV-5JS plasmid.
Fig. 5 shows the construction of the pM12 plasmid.
Fig. 6 shows the construction of the pM12-ter(thr) plasmid. The sequences in the drawing are shown as SEQ ID NOS: 24 and 25.
Fig. 7 shows the construction of the IntJS cassette.
Fig. 8 shows growth of a *d0191*-deficient strain and a *d0191*-enhanced strain of *P. ananatis* in the presence of L-cysteine.
Fig. 9 shows growth of a *d0191*-enhanced strain and a *d0191-*non-enhanced strain of *E. coli* in the presence of L-cysteine.
Fig. 10 shows results of cysteine desulfhydrase activity staining of *d0191* product.

### Detailed Description of the Preferred Embodiments

### <1> Bacterium

The bacterium belongs to the family *Enterobacteriaceae,* and is able to produce L-cysteineFurthermore, the bacterium has been modified to decrease the activity of the protein encoded by the *d0191* gene should be decreased. The *d0191* gene will be explained later.

The "ability to produce L-cysteine" or the "L-cysteine-producing ability" means an ability of the bacterium to produce and cause accumulation of L-cysteine in a medium or the bacterial cells in such an amount that the L-cysteine can be collected from the medium or cells when the bacterium is cultured in the medium. A bacterium having L-cysteine-producing ability means a bacterium which can produce and cause accumulation of a larger amount of L-cysteine as compared to a wild-type, parent, or unmodified strain, and preferably a bacterium which can produce and cause accumulation of L-cysteine in a medium in an amount of 0.3 g/L or more, more preferably 0.4 g/L or more, particularly preferably 0.5 g/L or more.

The L-cysteine produced by the bacterium may change into L-cystine in the medium by the formation of a disulfide bond. Furthermore, as described later, S-sulfocysteine may be generated by the reaction of L-cysteine and thiosulfuric acid in the medium (Szczepkowski T.W., Nature, vol. 182 (1958)). Furthermore, the L-cysteine generated in bacterial cells may be condensed with a ketone, aldehyde, or, for example, pyruvic acid, which is present in the cells, to produce a thiazolidine derivative via a hemithioketal intermediate (refer to Japanese Patent No. 2992010). This thiazolidine derivative and hemithioketal may be present as an equilibrated mixture. Therefore, the L-cysteine-producing ability is not limited to the ability to accumulate only L-cysteine in the medium or cells, but also includes the ability to accumulate, in addition to L-cysteine, L-cystine or derivatives thereof such as S-sulfocysteine, a thiazolidine derivative, or a hemithioketal or a mixture thereof in the medium.

The bacterium having L-cysteine-producing ability may have the ability to produce L-cysteine, or it may be imparted by modifying a microorganism such as those described below by mutagenesis or a recombinant DNA techniqueUunless specially mentioned, the term L-cysteine refers to the reduced-type L-cysteine, L-cystine, derivative such as those mentioned above, or a mixture thereof.

The bacterium is not particularly limited so long as the bacterium belongs to the family *Enterobacteriaceae* and has L- cysteine-producing ability. Such bacteria include those of the genera *Escherichia, Enterobacter, Pantoea, Klebsiella, Serratia, Erwinia, Salmonella* and *Morganella.* Specifically, those classified into the family *Enterobacteriaceae* according to the taxonomy used in the NCBI (National Center for Biotechnology Information) database
(http://www.ncbi.nlm.nih.gov/TaxonomyBrowser/wwwtax.cgi?id=91347) can be used. As the parent strain of the family *Enterobacteriaceae* which can be used to perform the modification, it is desirable to use, especially, a bacterium of the genus *Escherichia, Enterobacter, Pantoea, Erwinia,* or *Klebsiella.*

Although the *Escherichia* bacteria are not particularly limited, specifically, those described in the work of Neidhardt et al. (Backmann B.J., 1996, Derivations and Genotypes of some mutant derivatives of Escherichia coli K-12, p.2460-2488, Table 1, In F.D. Neidhardt (ed.), Escherichia coli and Salmonella Cellular and Molecular Biology/Second Edition, American Society for Microbiology Press, Washington, D.C.) can be used. *Escherichia coli* is preferable. Examples of *Escherichia coli* include *Escherichia coli* W3110 (ATCC 27325), *Escherichia coli* MG1655 (ATCC 47076), and so forth, and include those derived from the prototype wild-type strain, K12 strain.

These strains are available from, for example, American Type Culture Collection (Address: 12301 Parklawn Drive, Rockville, Maryland 20852, P.O. Box 1549, Manassas, VA 20108, United States of America). That is, registration numbers are given to each of the strains, and the strains can be ordered by using these registration numbers (refer to http://www.atcc.org/). The registration numbers of the strains are listed in the catalogue of the American Type Culture Collection.

Examples of the *Enterobacter* bacteria include *Enterobacter agglomerans, Enterobacter aerogenes* and so forth, and examples of the *Pantoea* bacteria include *Pantoea ananatis.* Some strains of *Enterobacter agglomerans* were recently reclassified into *Pantoea agglomerans, Pantoea ananatis,* or *Pantoea stewartii* on the basis of the nucleotide sequence analysis of 16S rRNA etc.. A bacterium belonging to either *Enterobacter* or *Pantoea* may be used so long as it is classified as the family *Enterobacteriaceae.*

In particular, *Pantoea* bacteria, *Erwinia* bacteria, and *Enterobacter* bacteria are classified as γ-proteobacteria, and they are taxonomically very close to one another (J. Gen. Appl. Microbiol., 1997, 43, 355-361; Int. J. Syst. Bacteriol., 1997, 43, 1061-1067). In recent years, some bacteria belonging to the genus *Enterobacter* were reclassified as *Pantoea agglomerans, Pantoea dispersa,* or the like, on the basis of DNA-DNA hybridization experiments etc. (International Journal of Systematic Bacteriology, July 1989, 39:337-345). Furthermore, some bacteria belonging to the genus *Erwinia* were reclassified as *Pantoea ananas* or *Pantoea stewartii* (refer to Int. J. Syst. Bacteriol., 1993, 43:162-173).

Examples of the *Enterobacter* bacteria include, but are not limited to, *Enterobacter agglomerans, Enterobacter aerogenes,* and so forth. Specifically, the strains exemplified in European Patent Publication No. 952221 can be used.

A typical strain of the genus *Enterobacter* is the *Enterobacter agglomeranses* ATCC 12287 strain.

Typical strains of the *Pantoea* bacteria include, but are not limited to, *Pantoea ananatis, Pantoea stewartii, Pantoea agglomerans,* and *Pantoea citrea.*

Specific examples of *Pantoea ananatis* include the *Pantoea ananatis* AJ13355 strain, SC 17 strain and SC 17(0) strain. The SC 17 strain was selected as a low phlegm-producing mutant strain from the AJ13355 strain (FERM BP-6614), which was isolated from soil in Iwata-shi, Shizuoka-ken, Japan for it's ability to proliferate in a low pH medium containing L-glutamic acid and a carbon source (U.S. Patent No. 6,596,517). The SC 17(0) strain was constructed as a strain resistant to the λ Red gene product for performing gene disruption in *Pantoea ananatis* (refer to Reference Example 1). The *Pantoea ananatis* AJ13355 strain was deposited at the National Institute of Bioscience and Human-Technology, Agency of Industrial Science and Technology, Ministry of International Trade and Industry (currently, the National Institute of Advanced Industrial Science and Technology, International Patent Organism Depositary, Address: Tsukuba Central 6, 1-1, Higashi 1-Chome, Tsukuba-shi, Ibaraki-ken, 305-8566, Japan) on February 19, 1998 and assigned an accession number of FERM P-16644. It was then converted to an international deposit under the provisions of Budapest Treaty on January 11, 1999 and assigned an accession number of FERM BP-6614. This strain was identified as *Enterobacter agglomerans* when it was isolated and deposited as the *Enterobacter agglomerans* AJ13355 strain. However, it was recently reclassified as *Pantoea ananatis* on the basis of nucleotide sequencing of 16S rRNA and so forth.

The SC17 strain was deposited at the National Institute of Advanced Industrial Science and Technology, International Patent Organism Depositary on February 4, 2009, and assigned the receipt number FERM BP-11091.

Examples of the *Erwinia* bacteria include, but are not limited to, *Erwinia amylovora* and *Erwinia carotovora,* and examples of the *Klebsiella* bacteria include *Klebsiella planticola.*

Hereinafter, methods for imparting L-cysteine-producing ability to bacteria belonging to *Enterobacteriaceae,* or methods for enhancing L-cysteine-producing ability of such bacteria, are described.

To impart the ability to produce L-cysteine, methods conventionally employed in the breeding of coryneform bacteria or bacteria of the genus *Escherichia* (see "amino Acid Fermentation", Gakkai Shuppan Center (Ltd.), 1st Edition, published May 30, 1986, pp. 77-100) can be used. Such methods include by acquiring the properties of an auxotrophic mutant, an analogue-resistant strain, or a metabolic regulation mutant, or by constructing a recombinant strain so that it overexpresses an L-cysteine biosynthesis enzyme. Here, in the breeding of an L-cysteine-producing bacteria, one or more of the above described properties may be imparted. The expression of L-cysteine biosynthesis enzyme(s) can be enhanced alone or in combinations of two or more. Furthermore, imparting properties such as an auxotrophic mutation, analogue resistance, or metabolic regulation mutation may be combined with the methods of enhancing the biosynthesis enzymes.

An auxotrophic mutant strain, L-cysteine analogue-resistant strain, or metabolic regulation mutant strain with the ability to produce L-cysteine can be obtained by subjecting a parent, wild-type, or unmodified strain to conventional mutatagenesis, such as exposure to X-rays or UV irradiation, or by treating with a mutagen such as N-such as exposure to X-rays or UV irradiation, or by treating with a mutagen such as N-methyl-N'-nitro-N-nitrosoguanidine, etc., then selecting those which exhibit autotrophy, analogue resistance, or a metabolic regulation mutation and which also have the ability to produce L-cysteine.

Specific examples of L-cysteine-producing bacteria include, but are not limited to, *E. coli* JM 15 transformed with multiple kinds of cysE gene alleles encoding serine acetyltransferase resistant to feedback inhibition (US Patent 6,218,168), *E. coli* W3110 in which a gene encoding a protein responsible for excretion of cytotoxic substances is overexpressed (U.S. Patent No. 5,972,663), an *E. coli* strain having decreased cysteine desulfhydrase activity (Japanese Patent Laid-open No. 11-155571), and *E. coli* W3110 with increased activity of the positive transcriptional control factor of the cysteine regulon encoded by the *cysB* gene is increased (WO01/27307).

The bacterium of the present invention has been modified to decrase the activity of the protein encoded by the *d0191* gene. As described later, the protein has cysteine desulfhydrase activity. The following proteins are known to have the cysteine desulfhydrase activity of *E. coli,* cystathionine-β-lyase (*metC* product, Japanese Patent Laid-open No. 11-155571, Chandra et al., Biochemistry, 21 (1982) 3064-3069), tryptophanase (*tnaA* product, Japanese Patent Laid-open No. 2003-169668, Austin Newton et al., J. Biol. Chem., 240 (1965) 1211-1218)), O-acetylserine sulfhydrylase B (*cysM* gene product, Japanese Patent Laid-open No. 2005-245311) and the *malY* gene product (Japanese Patent Laid-open No. 2005-245311) are known. In addition to the activity of the protein encoded by the *d0191* gene, the activities of these proteins may be decreased.

The L-cysteine-producing bacterium preferably has a SAT which has been mutated to be resistant to feedback inhibition. The following mutation in SAT are known to induce resistance to feedback inhibition and are derived from *Escherichia coli:* when the methionine residue at position 256 is replaced with a glutamate residue (Japanese Patent Laid-open No. 11-155571), when the methionine residue at position 256 is replaced with an isoleucine residue (Denk, D. and Boeck, A., J. General Microbiol., 133, 515-525 (1987)), a mutation in the region from the amino acid residue at position 97 to the amino acid residue at position 273 or a deletion of the C-terminus region from the amino acid residue at position 227 (International Patent Publication WO97/15673, U.S. Patent No. 6,218,168), when the amino acid sequence corresponding to positions 89 to 96 of wild-type SAT contains one or more mutations (U.S. Patent Published Application No. 20050112731(A1)) and so forth. In the *cysE5* gene which encodes the mutant SAT described in the examples, the Val residue and the Asp residue at positions 95 and 96 of the wild-type SAT are replaced with an Arg residue and Pro residue, respectively.

The SAT gene is not limited to the gene of *Escherichia coli,* but can be any gene encoding a protein having the SAT activity. An SAT isozyme of *Arabidopsis thaliana* and desensitized to feedback inhibition by L-cysteine is known, and the gene encoding this SAT can also be used (FEMS Microbiol. Lett., 179 (1999) 453-459).

If a gene encoding a mutant SAT is introduced into a bacterium, L-cysteine-producing ability is imparted to the bacterium. To introduce a mutant SAT gene into a bacterium, various vectors which are typically used for protein expression can be used. Examples of such vectors include pUC 19, pUC 18, pHSG299, pHSG399, pHSG398, RSF1010, pBR322, pACYC184, pMW219 and so forth.

In order to introduce a recombinant vector containing a SAT gene into a bacterium, methods which are typically used to transform bacteria can be used, such as the method of D.A. Morrison (Methods in Enzymology, 68, 326 (1979)), treating recipient cells with calcium chloride to increase permeability of the cells for DNA (Mandel, M. and Higa, A., J. Mol. Biol., 53, 159 (1970)), and a method based on electroporation.

Furthermore, the SAT activity can also be enhanced by increasing the copy number of the SAT gene. The copy number of the SAT gene can be increased by introducing the SAT gene into a bacterium by using a vector such as those described above, or by introducing multiple copies of the SAT gene onto the chromosomal DNA of a bacterium. Multiple copies of the SAT gene are introduced by homologous recombination which targets a sequence present on the chromosomal DNA in multiple copies. A repetitive DNA or inverted repeat present at the end of a transposable element can be used as a sequence which is present on the chromosomal DNA in multiple copies. Alternatively, as disclosed in Japanese Patent Laid-open No. 2-109985, multiple copies of the SAT gene can be introduced into the chromosomal DNA by incorporating them into a transposon and transferring it.

The bacterium can be obtained by modifying a bacterium which belongs to the family *Enterobacteriaceae* and which has L-cysteine-producing ability and the *d0191* gene such as those described above, so that the activity of the protein encoded by the *d0191* gene (henceforth also referred to as "D0191") is decreased. Alternatively, after such modification that the activity of the D0191 protein is decreased, L-cysteine-producing ability may be imparted.

The inventor of the present invention found a novel gene encoding a protein having cysteine desulfhydrase activity from the chromosomal DNA of *Pantoea ananatis,* and designated it as *d0191* gene. The nucleotide sequence of this gene and the amino acid sequence encoded by the gene are shown in SEQ ID NOS: 1 and 2, respectively.

When databases were searched for the sequence of the *d0191* gene of *Pantoea ananatis,* genes considered to be homologue genes of the *d0191* gene of which functions were unknown were found in the following bacteria. The nucleotide sequences of these genes and the amino acid sequences encoded by those nucleotide sequences are shown in SEQ ID NOS: 34 to 52. Accession numbers in the NCBI (National Center for Biotechnology Information) database are shown in the parentheses (GenBank Identifier(gi)|RefSeq accession (ref)).

*Citrobacter koseri* ATCC BAA-895 (accession: gi|157146936|ref|YP_001454255.1|)

*Klebsiella pneumoniae* subsp. pneumoniae MGH 78578 (accession: gi|152968982|ref|YP_001334091.1|)

*Enterobacter* sp. 638 (accession: gi|146310575|ref|YP_001175649.1|)

*Salmonella typhimurium* LT2 (accession: gi|16763839|ref|NP_459454.1|) *Serratia proteamaculans* 568 (accession: gi|157369348|ref|YP_001477337.1|)

*Erwinia carotovora* subsp. atroseptica SCRI1043 (accession: gi|50120095|ref|YP_049262.1|)

*vibrio cholerae* O1 biovar eltor str. N16961 (accession: gi|15641074|ref|NP_230706.1|)

*Pseudomonas fluorescens* PfO-1 (accession: gi|77457462|ref|YP_346967.1|)

*Streptomyces coelicolor* A3(2) (accession: gi|21219544|ref|NP_625323.1|)

*Mycobacterium avium* 104 (accession: gil|118467280|ref|YP_879726.1|)

In the present invention, the *d0191* gene of *Pantoea ananatis* and homologue genes of this gene possessed by other bacteria may be called *d0191* gene.

The phrase "decrease the activity of the protein encoded by the *d0191* gene" means that the activity of the D0191 protein encoded by the *d0191* gene is decreased as compared to a non-modified strain such as a wild-type strain or parent strain, and also means the complete disappearance of the activity.

As described in the examples, it was demonstrated that the protein encoded by the *d0191* gene, i.e., the D0191 protein, had the cysteine desulfhydrase activity by activity staining. Therefore, the activity of the D0191 protein specifically means the cysteine desulfhydrase activity. The cysteine desulfhydrase activity can be measured by, for example, the method described in Japanese Patent Laid-open No. 2002-233384.

Modifications to decrease the activity of the D0191 proteinare attained by, for example, reducing the expression of the *d0191* gene. Specifically, for example, intracellular activity of the protein can be reduced by deleting a part or all of the coding region of the *d0191* gene on the chromosome. Furthermore, the activity of the D0191 protein can also be decreased by reducing the expression, for example, by modifying an expression control sequence of the gene such as a promoter or the Shine-Dalgarno (SD) sequence. Furthermore, the expression amount of the gene can also be reduced by modifying a non-translated region other than the expression control sequence. Furthermore, the whole gene as well as the sequences on both sides of the gene on the chromosome may be deleted. Furthermore, mutations which cause an amino acid substitution (missense mutation), a stop codon (nonsense mutation), or a frame shift mutation which adds or deletes one or two nucleotides into the coding region of the *d0191* gene on the chromosome can be introduced (Journal of Biological Chemistry, 272:8611-8617 (1997); Proceedings of the National Academy of Sciences, USA, 95 5511-5515 (1998); Journal of Biological Chemistry, 266, 20833-20839 (1991)).

Furthermore, a transcriptional regulator, *ybaO* gene (synonym: c0263), is present upstream of the d0191 gene. The activity of the D0191 protein can be decreased by inactivating the *ybaO* gene. The nucleotide sequence of the *ybaO* gene from *Pantoea ananatis* and the amino acid sequence encoded by the gene are shown in SEQ ID NOS: 66 and 67, respectively.

Furthermore, modification can be caused by a conventional mutagenesis based on X-ray or ultraviolet irradiation or the use of a mutagen such as N-methyl-N'-nitro-N-nitrosoguanidine, so long as the activity of the D0191 protein is decreased.

An expression control sequence can be modified for preferably one or more nucleotides, more preferably two or more nucleotides, particularly preferably three or more nucleotides. When a coding region is deleted, the region to be deleted may be an N-terminus region, an internal region or a C-terminus region, or even the entire coding region, so long as the function of the D0191 protein is decreased or deleted. Deletion of a longer region can usually more surely inactivate a gene. Further, it is preferred that the deleted reading frames upstream and downstream of the region are not the same.

When another sequence is inserted into the coding region of the *d0191* gene, the sequence may be inserted into any region of the gene, and insertion of a longer sequence can usually more surely inactivate the gene. It is preferred that the reading frames upstream and downstream of the insertion site are not the same. The other sequence is not particularly limited so long as a sequence which decreases or deletes function of the encoded D0191 protein is chosen, and examples include a transposon carrying an antibiotic resistance gene, a gene useful for L-cysteine production and so forth.

The *d0191* gene on the chromosome can be modified as described above by, for example, preparing a deletion-type version of the gene in which a partial sequence of the gene is deleted so that the deletion-type gene does not produce normally-functioning D0191 proteins, and transforming a bacterium with a DNA containing the deletion-type gene to cause homologous recombination between the deletion-type gene and the native gene on the chromosome, which results in the substitution of the deletion-type gene for the gene on the genome. The D0191 protein encoded by the deletion-type gene has a conformation different from that of the wild-type enzyme protein, if it is even produced, and thus the function is reduced or deleted. Such gene disruption based on gene substitution utilizing homologous recombination is known, and examples includes Red-driven integration (Datsenko, K.A, and Wanner, B.L., Proc. Natl. Acad. Sci. USA, 97:6640-6645 (2000)), using a linear DNA in Red driven integration in combination with an excisive system derived from λ phage (Cho, E.H., Gumport, R.I., Gardner, J.F., J. Bacteriol., 184:5200-5203 (2002)), using a plasmid containing a temperature sensitive replication origin or a plasmid capable of conjugative transfer, utilizing a suicide vector without a replication origin in a host (U.S. Patent No. 6,303,383, Japanese Patent Laid-open No. 05-007491), and so forth.

Decrease of the expression of the *d0191* gene can be confirmed by comparing the amount of mRNA transcribed from the gene with that in a wild-type strain or unmodified strain. The expression amount can be confirmed by Northern hybridization, RT-PCR (Sambrook, J., Fritsch, E. F., and Maniatis, T. 1989, Molecular Cloning A Laboratory Manual/Second Edition, Cold Spring Harbor Laboratory Press, New York.), and the like.

Decrease of the amount of D0191 protein can be confirmed by Western blotting using antibodies (Molecular cloning, Cold spring Harbor Laboratory Press, Cold spring Harbor, USA, 2001).

Decrease of the amount of the D0191 protein can also be confirmed by measuring the cysteine desulfhydrase activity of cell.

Since the nucleotide sequence of the *d0191* gene may different depending on species or strain to which a bacterium belongs, the *d0191* gene to be modified may be a variant of the nucleotide sequence of SEQ ID NO: 1. Moreover, the *d0191* gene to be modified may be any of the aforementioned *d0191* gene homologues, for example, a variant of a gene having the nucleotide sequence shown as SEQ ID NOS: 33, 35, 37, 39, 41, 43, 45, 47, 49 or 51.

Variant of the *d0191* gene can be searched for with BLAST (http://blast.genome.jp/) or the like by referring to the nucleotide sequence of SEQ ID NO: 1 or SEQ ID NO: 33, 35, 37, 39, 41, 43, 45, 47, 49 or 51. Moreover, variants of the *d0191* gene include genes which can be amplified by PCR using a chromosome of bacterium belonging to the family *Enterobacteriaceae* or the like as a template, and synthetic oligonucleotides prepared on the basis of the nucleotide sequence of SEQ ID NO: 1 or SEQ ID NO: 33, 35, 37, 39, 41, 43, 45, 47, 49 or 51.

Moreover, the *d0191* gene may also be a gene encoding a protein having a sequence corresponding to the amino acid sequence of the D0191 protein, such as a sequence of SEQ ID NO: 2 or SEQ ID NO: 34, 36, 38, 40, 42, 44, 46, 48, 50 or 52, including substitutions, deletions, insertions, additions or the like of one or several amino acid residues at one or several positions. Although the number of the "one or several" amino acid residues may differ depending on their position in the three-dimensional structure or the types of amino acid residues of the proteins, it is preferably 1 to 20, more preferably 1 to 10, particularly preferably 1 to 5. These substitutions, deletions, insertions, or additions of one or several amino acids are preferably conservative mutations so that the function of the protein is maintained. A conservative mutation is a mutation wherein substitution takes place mutually among Phe, Trp and Tyr, if the substitution site is an aromatic amino acid; among Leu, Ile and Val, if the substitution site is a hydrophobic amino acid; between Gln and Asn, if it is a polar amino acid; among Lys, Arg and His, if it is a basic amino acid; between Asp and Glu, if it is an acidic amino acid; and between Ser and Thr, if it is an amino acid having a hydroxy group. Typical examples of conservative mutations are conservative substitutions. Specific examples of conservative substitutions include: substitution of Ser or Thr for Ala; substitution of Gln, His or Lys for Arg; substitution of Glu, Gln, Lys, His or Asp for Asn; substitution of Asn, Glu or Gln for Asp; substitution of Ser or Ala for Cys; substitution of Asn, Glu, Lys, His, Asp or Arg for Gln; substitution of Gly, Asn, Gin, Lys or Asp for Glu; substitution of Pro for Gly; substitution of Asn, Lys, Gln, Arg or Tyr for His; substitution of Leu, Met, Val or Phe for Ile; substitution of Ile, Met, Val or Phe for Leu; substitution of Asn, Glu, Gln, His or Arg for Lys; substitution of Ile, Leu, Val or Phe for Met; substitution of Trp, Tyr, Met, Ile or Leu for Phe; substitution of Thr or Ala for Ser; substitution of Ser or Ala for Thr; substitution of Phe or Tyr for Trp; substitution of His, Phe or Trp for Tyr; and substitution of Met, Ile or Leu for Val. The above-mentioned amino acid substitution, deletion, insertion, addition, inversion, etc. may be the result of a naturally-occurring mutation or variation due to an individual difference, or a difference of species of a bacterium.

The *d0191* gene provided by the present invention as a novel gene encodes a protein having the amino acid sequence of SEQ ID NO: 2, or a protein having an amino acid sequence of SEQ ID NO: 2 including substitutions, deletions, insertions or additions of 1 to 50, preferably 1 to 40, more preferably 1 to 30, still more preferably 1 to 20, further still more preferably 1 to 10, particularly preferably 1 to 5, of amino acid residues, and having the cysteine desulfhydrase activity.

Furthermore, the gene having such a conservative mutation as mentioned above may be a gene encoding a protein showing a homology of 80% or more, preferably 90% or more, more preferably 95% or more, still more preferably 97% or more, further still more preferably 98% or more, particularly preferably 99% or more, to the entire encoded amino acid sequence, and which has a function equivalent to that of a wild-type D0191 protein. In the present specification, the term "homology" may mean "identity".

The *d0191* gene may be a DNA which hybridizes with a probe prepared from known gene sequences, for example, the above described gene sequences or sequences complementary to the sequences under stringent conditions and which encodes a protein which is a functional equivalent to the D0191 protein. The term "stringent conditions" refers to conditions where a so-called specific hybrid is formed and a non-specific hybrid is not formed. Examples thereof include conditions where DNAs having high homology, for example, at least 80%, preferably 90%, more preferably 95%, more preferably 97%, more preferably 98%, further preferably 99% homology, hybridize with each other and DNAs having homology less than the value do not hybridize with each other; and specifically include conditions corresponding to a salt concentration and temperature of washing which are typical of Southern hybridization, e.g., washing at 60°C, 1×SSC, 0.1% SDS, preferably 60°C, 0.1×SSC, 0.1% SDS, more preferably 68°C, 0.1×SSC, 0.1 % SDS, once or preferably twice or three times.

The probe may be a partial sequence of the gene. Such a probe can be prepared by PCR using oligonucleotides prepared based on the known nucleotide sequences of genes as primers, and a DNA fragment containing these sequences as the template. When a DNA fragment of a length of about 300 bp is used as the probe, the conditions of washing after hybridization can be, for example, 50°C, 2×SSC, and 0.1% SDS.

The above descriptions about variants of genes and proteins are similarly applied to enzymes such as serine acetyltransferase and genes coding for them.

### <2> Method for producing L-cysteine, L-cystine, derivative thereof or mixture thereof

These compounds can be produced by culturing the bacterium obtained as described above in a medium, and collecting L-cysteine, L-cystine, a derivative thereof or a mixture thereof from the medium. Examples of a derivative of L-cysteine include S-sulfocysteine, a thiazolidine derivative, a hemithioketal corresponding the thiazolidine derivative mentioned above and so forth.

Examples of the medium used for the culture include ordinary media containing a carbon source, nitrogen source, sulfur source, inorganic ions, and other organic components as required.

As the carbon source, saccharides such as glucose, fructose, sucrose, molasses and starch hydrolysate, and organic acids such as fumaric acid, citric acid and succinic acid can be used.

As the nitrogen source, inorganic ammonium salts such as ammonium sulfate, ammonium chloride and ammonium phosphate, organic nitrogen such as soybean hydrolysate, ammonia gas, aqueous ammonia and so forth can be used.

As the sulfur source, inorganic sulfur compounds, such as sulfates, sulfites, sulfides, hyposulfites and thiosulfates can be used.

As organic trace amount nutrients, it is desirable to add required substances such as vitamin B₁, yeast extract, and so forth in appropriate amounts. Other than these, potassium phosphate, magnesium sulfate, iron ions, manganese ions and so forth are added in small amounts.

The culture is preferably performed under aerobic conditions for 30 to 90 hours. The culture temperature is preferably controlled to be 25°C to 37°C, and the pH is preferably controlled to be 5 to 8 during the culture. To adjust the pH, inorganic or organic acidic or alkaline substances, ammonia gas and so forth can be used. Collection of L-cysteine from the culture can be attained by, for example,any combination of known ion exchange resin method, precipitation and other known methods.

L-cysteine obtained as described above can be used toproduce L-cysteine derivatives. The cysteine derivatives include methylcysteine, ethylcysteine, carbocysteine, sulfocysteine, acetylcysteine, and so forth.

Furthermore, when a thiazolidine derivative of L-cysteine is produced in the medium, L-cysteine can be produced by collecting the thiazolidine derivative from the medium to break the reaction equilibrium between the thiazolidine derivative and L-cysteine so that L-cysteine should be excessively produced.

Furthermore, when S-sulfocysteine is produced in the medium, it can be converted into L-cysteine by reduction with a reducing agent such as dithiothreitol.

### Examples

Hereinafter, the present invention will be explained more specifically with reference to the following non-limiting examples.

### Reference Example 1: Construction of Pantoea ananatis strain resistant to λ Red gene product

In order to perform gene disruption in *Pantoea ananatis,* a recipient strain for efficiently carrying out the method called "Red-driven integration" or "Red-mediated integration" (Proc. Natl. Acad. Sci. USA., 97, 6640-6645 (2000)) was constructed.

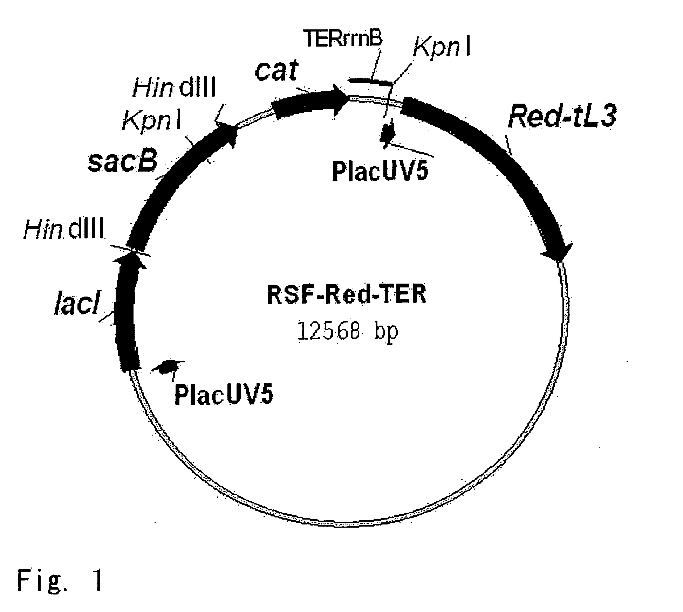

First, a novel helper plasmid RSF-Red-TER which expresses the *gam, bet* and *exo* genes of λ (henceforth referred to as "λ Red gene") was constructed (Fig. 1). The details are described in Reference Example 2.

This plasmid can be used for a wide range of hosts of different genetic backgrounds. This is because 1) this plasmid has the replicon of the RSF1010 wide host spectrum plasmid (Scholz, et al., 1989; Buchanan-Wollaston et al., 1987), which may be stably maintained by many gram negative and gram positive bacteria, and even plants, 2) the λ Red gene, *gam, bet* and *exo* genes, are under the control of the P_{lacUV5} promoter, which is recognized by RNA polymerases of many bacteria (for example, Brunschwig, E. and Darzins, A., Gene, 111, 1, 35-41 (1992); Dehio, M. et al, Gene, 215, 2, 223-229 (1998)), and 3) the autoregulation factor P_{lacUC5}-lacI and the ρ-non-dependent transcription terminator (TrrnB) of the *rrnB* operon of *Escherichia coli* lower the basal expression level of the λ Red gene (Skorokhodova, A. Yu et al, Biotekhnologiya (Rus), 5, 3-21 (2004)). Furthermore, the RSF-Red-TER plasmid contains the levansucrase gene *(sacB),* and by using this gene, the plasmid can be collected from cells in a medium containing sucrose.

In *Escherichia coli,* frequency of integration of a DNA fragment generated by PCR together with the short flanking region provided by the RSF-Red-TER plasmid is high to the same extent as that in the case where the pKD46 helper plasmid (Datsenko, KA., Wanner, B.L., Proc. Natl. Acad. Sci. USA, 97, 6640-6645 (2000)) is used. However, expression of the λ Red gene is toxic for *Pantoea ananatis.* The cells transformed with the RSF-Red-TER helper plasmid extremely slowly grew in the LB medium containing IPTG (isopropyl-β-D-thiogalactopyranoside, 1 mM) and an appropriate antibiotic (25 µg/ml of chloramphenicol or 40 µg/ml of kanamycin), and the efficiency of λ Red-mediated recombination is extremely low (10⁻⁸), even if it is observed.

A variant strain of *Pantoea ananatis* resistant to expression of all the three genes of λ Red gene was selected. For this purpose, the RSF-Red-TER plasmid was introduced into the *Pantoea ananatis* SC17 strain (U.S. Patent No. 6,596,517) by electroporation. After culture of 18 hours, about 10⁶ of transformants were obtained, 10 clones among them formed colonies of large size, and all the remainder formed extremely small colonies. After culture of 18 hours, the large colonies had a size of about 2 mm, and the small colonies had a size of about 0.2 mm. Whereas the small colonies did not grow any more even if the culture was extended until 24 hours, the large colonies continued to grow. One of the *Pantoea ananatis* variant strains forming large colonies, which was resistant to expression of all the three genes of λ Red gene

(*gam, bet,* and *exo*)*,* was used for the further analysis.

The RSF-Red-TER plasmid DNA was isolated from one clone among those of the large colonies and several clones among those of small colonies, and transformed again into *Escherichia coli* MG1655 to examine the ability of the plasmid to synthesize an active Red gene product. By a control experiment for Red-dependent integration in the obtained transformants, it was demonstrated that only the plasmid isolated from the clone of the large colony induced expression of the λ Red gene required for the Red-dependent integration. In order to investigate whether the Red-mediated integration occurs in the selected clone of the large colony, electroporation was performed by using a linear DNA fragment produced by PCR, which was designed so that it contains a Km^{R} marker and a flanking region of 40 bp homologous to the *hisD* gene, and it is integrated in the *hisD* gene of *Pantoea ananatis* at the *SMaI* recognition site. Two clones of small colonies were used as control. The nucleotide sequence of the *hisD* gene *of Pantoea ananatis* is shown in SEQ ID NO: 3. For PCR, the oligonucleotides of SEQ ID NOS: 4 and 5 were used as primers, and a pMW118-(λatt-Km^{r}-λatt) plasmid was used as a template. The two clones of small colonies, which were not resistant to the λ Red gene, were used as control. Construction of the pMW118-(λattL-Kmr-λattR) plasmid will be explained in detail in Reference Example 3.

The RSF-Red-TER plasmid can induce expression of the Red gene with the *lacI* gene carried on the plasmid. Two kinds of induction conditions were investigated. In the first group, IPTG (1 mM) was added 1 hour before the electroporation, and in the second group, IPTG was added at the start of the culture for preparation of cells of which electroporation is possible. The growth rate of the progeny of the cells harboring RSF-Red-TER derived from the clone of large colony was not significantly lower than that of a strain not harboring the SC17 plasmid. The addition of IPTG only slightly decreased the growth rate of these cultures. On the other hand, the progeny of the clones of small colonies extremely slowly grew even without addition of IPTG, and after induction, growth was substantially arrested. After electroporation of the cells of the progeny of the clone of large colony, many Km^{R} clones grew (18 clones after a short induction time, and about 100 clones after extended induction time). All the 100 clones investigated had a His phenotype, and about 20 clones were confirmed by PCR to have the expected structure of chromosome in the cells thereof. On the other hand, even when electroporation was performed for the cells of the progeny of the clones of small colonies, any integrated strain could not be obtained.

The obtained clone of large colony was grown on a plate containing 7% sucrose to eliminate the plasmid, and transformed again with RSF-Red-TER. A strain not harboring the plasmid was designated SC 17(0). This strain was deposited at the Russian National Collection of Industrial Microorganisms (VKPM, GNII Genetica, address: Russia, 117545 Moscow, 1 Dorozhny proezd. 1) on September 21, 2005, and assigned an accession number of VKPM B-9246.

All the clones grown after the aforementioned re-transformation showed large colony sizes like the parent strain clone SC 17(0). The Red-mediated integration experiment was performed in the SC17(0) strain re-transformed with the RSF-Red-TER plasmid. Three of the obtained independent transformants were investigated by using the same DNA fragment as that used for the previous experiment. The short induction time (1 hour before electroporation) was employed. Km^{R} Clones exceeding ten clones grew in each experiment. All the examined clones had the His phenotype. In this way, a variant strain designated SC 17(0) resistant to the expression of the λ Red gene was selected. This strain can be used as a recipient strain suitable for the Red-dependent integration into the *Pantoea ananatis* chromosome.

### Reference Example 2: Construction of helper plasmid RSF-Red-TER

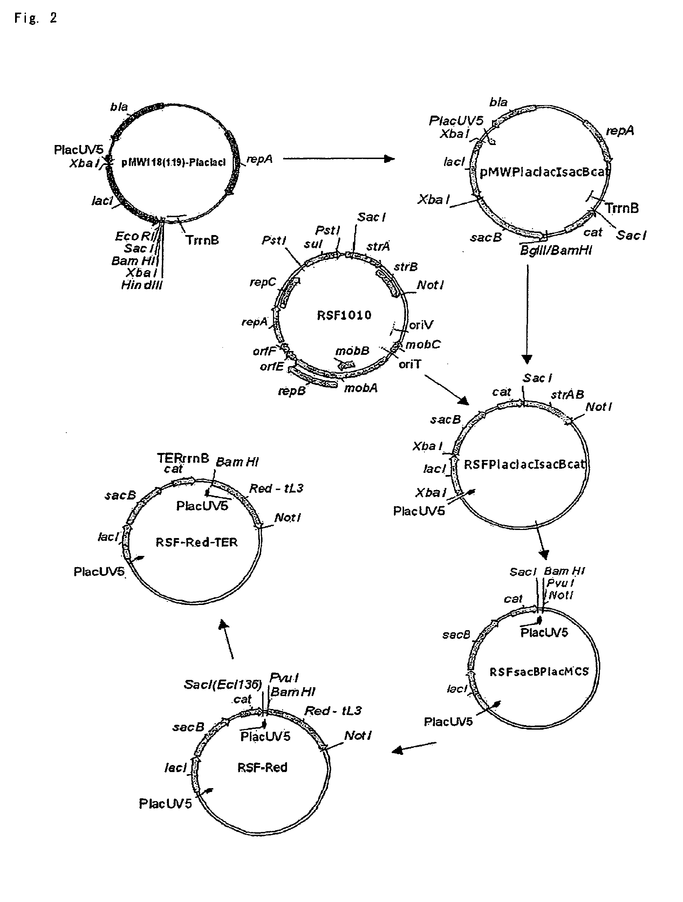

The construction scheme of the helper plasmid RSF-Red-TER is shown in Fig. 2.

As a first step of the construction, a RSFsacBPlacMCS vector was designed. For this purpose, DNA fragments containing the *cat* gene of pACYC184 plasmid and the structural gene region of the *sacB* gene of *Bacillus subtilis* were amplified by PCR using oligonucleotide of SEQ ID NOS: 6 and 7, and 8 and 9, respectively. These oligonucleotides contained convenient *Bgl*II*, Sac*I*, Xba*I and *Bam*HI restriction enzyme sites required for further cloning in the 5' end regions, respectively. The obtained *sacB* fragment of 1.5 kb was cloned into the previously obtained pMW119-P_{lac}lacI vector at the *Xba*I*-Bam*HI site. This vector was constructed in the same manner as that described for the pMW118-P_{lac}lacI vector (Skorokhodova, A. Yu et al, Biotekhnologiya (Rus), 5, 3-21 (2004)). However, this vector contained a polylinker moiety derived from pew219 instead of the pMW218 plasmid.

Then, the aforementioned *cat* fragment of 1.0 kb was treated with *Bgl*II and *SacI,* and cloned into the RSF-P_{lac}lacIsacB plasmid obtained in the previous step at the BamHI-SacI site. The obtained plasmid pMW-P_{lac}lacIsacBcat contained the PlacUV5-lacI-sacB-cat fragment. In order to subclone this fragment into the RSF1010 vector, PMW-P_{lac}lacIsacBcat was digested with *Bgl*II*,* blunt-ended by a treatment with DNA polymerase I Klenow fragment, and successively digested with *Sac*I*.* A 3.8 kb *Bgl*II-*Sac*I fragment of the pMWP_{lac}lacIsacBcat plasmid was eluted from 1% agarose gel, and ligated with the RSF1010 vector treated with *Pst*I and *Sac*I*. Escherichia coli* TG1 was transformed with the ligation mixture, and plated on the LB medium containing chloramphenicol (50 mg/L). The plasmids isolated from the grown clones were analyzed with restriction enzymes to obtain an RSFsacB plasmid. In order to construct a RSFsacBP_{lac}MCS vector, a DNA fragment containing the P_{lacUV5} promoter was amplified by PCR using oligonucleotides of SEQ ID NOS: 10 and 11 as primers and the pMW 119-P_{lac}lacI plasmid as a template. The obtained fragment of 146 bp was digested with *Sac*I and *Not*I, and ligated with the *Sac*I*-Not*I large fragment of the RSFsacB plasmid. Then, by PCR using the oligonucleotides of SEQ ID NOS: 12 and 13 as primers, and the pKD46 plasmid (Datsenko, KA., Wanner, B.L., Proc. Natl. Acad. Sci. USA, 97, 6640-6645 (2000)) as a template, a DNA fragment of 2.3 kb containing the λRedαβγ gene and the transcription terminator tL3 was amplified. The obtained fragment was cloned into the RSFsacBP_{lac}MCS vector at the *Pvu*I*-Not*I site. In this way, the RSFRed plasmid was designed.

In order to eliminate the read through transcription of the Red gene, a p-dependent transcription terminator of the *rrnB* operon of *Escherichia coli* was inserted at a position between the *cat* gene and the P_{lacUV5} promoter. For this purpose, a DNA fragment containing the P_{lacUV5} promoter and the TrrnB terminator was amplified by PCR using the oligonucleotides of SEQ ID NOS: 14 and 11 as primers and the chromosome of *Escherichia coli* BW3350 as a template. These obtained fragments were treated with *Kpn*I and ligated. Then, the 0.5 kb fragment containing both P_{lacUV5} and TrrnB was amplified by PCR using the oligonucleotides of SEQ ID NOS: 11 and 15 as primers. The obtained DNA fragment was digested with EcoRI, blunt-ended by a treatment with DNA polymerase I Klenow fragment, digested with *Bam*HI*,* and ligated with the *Ecl*136II*-Bam*HI large fragment of the RSFsacBPlacMCS vector. The obtained plasmid was designated RSF-Red-TER.

Reference Example 3: Construction of the pMW118-(λ*attL*-Km^{r}-λ*attR*) plasmid.

The pMW118-(λattL-Km^{r}-λ*attR*) plasmid was constructed based on the pMW 118-attL-Tc-attR (W02005/010175) plasmid by substitution of the tetracycline resistance marker gene with the kanamycin resistance marker gene from the pUC4K plasmid (Vieira, J. and Messing, J., Gene, 19(3): 259-68 (1982)). For that purpose, the large *Eco*RI*-Hind*III fragment from pMW118-attL-Tc-attR plasmid was ligated to two fragments from the pUC4K plasmid: *Hind*III *- Pst*I fragment (676 bp) and *Eco*RI-*Hind*III fragment (585 bp). Basic pMW118-attL-Tc-attR was obtained by ligation of the following four DNA fragments:
1) the *Bgl*II*-EcoR*I fragment (114 bp) carrying *attL* (SEQ ID NO: 26) which was obtained by PCR amplification of the corresponding region of the *E. coli* W3350 (contained λ prophage) chromosome using oligonucleotides P1 and P2 (SEQ ID NOS: 53 and 54) as primers (these primers contained the subsidiary recognition sites for *Bgl*II and *EcoR*I endonucleases);
2) the *Pst*I*-Hind*III fragment (182 bp) carrying *attR* (SEQ ID NO: 58) which was obtained by PCR amplification of the corresponding region of the *E. coli* W3350 (contained λ prophage) chromosome using the oligonucleotides P3 and P4 (SEQ ID NOS: 56 and 57) as primers (these primers contained the subsidiary recognition sites for *Pst*I and *Hind*III endonucleases);
3) the large *Bgl*II*-Hind*III fragment (3916 bp) of pMW118-ter_*rrn*B. The plasmid pMW118-ter_*rrnB* was obtained by ligation of the following three DNA fragments:
   a) the large DNA fragment (2359 bp) carrying the *Aat*II*-EcoR*I fragment of pMW118 that was obtained by the following way: pMW118 was digested with *EcoR*I restriction endonuclease, treated with Klenow fragment of DNA polymerase I, and then digested with *Aat*II restriction endonuclease;
   b) the small AatII-*Bgl*II fragment (1194 bp) of pUC19 carrying the *bla* gene for ampicillin resistance (Ap^{R}) was obtained by PCR amplification of the corresponding region of the pUC 19 plasmid using oligonucleotides P5 and P6 (SEQ ID NOS: 59 and 60) as primers (these primers contained the subsidiary recognition sites for *Aat*II and *Bgl*II endonucleases);
   c) the small *Bgl*II*-Pst*Ipol fragment (363 bp) of the transcription terminator ter_rrnB was obtained by PCR amplification of the corresponding region of the *E. coli* MG1655 chromosome using oligonucleotides P7 and P8 (SEQ ID NOS: 61 and 62) as primers (these primers contained the subsidiary recognition sites for *Bgl*II and *PstI* endonucleases);
4) the small *Eco*RI*-Pst*I fragment (1388 bp) (SEQ ID NO: 63) ofpML-Tc-ter_*thrL* bearing the tetracycline resistance gene and the ter_*thrL* transcription terminator; the pML-Tc-ter_*thrL* plasmid was obtained in two steps:
   - the pML-ter_*thrL* plasmid was obtained by digesting the pML-MCS plasmid (Mashko, S.V. et al., Biotekhnologiya (in Russian), 2001, no. 5, 3-20) with the *XbaI* and *BamH*I restriction endonucleases, followed by ligation of the large fragment (3342 bp) with the *Xba*I*-BamH*I fragment (68 bp) carrying terminator ter_*thrL* obtained by PCR amplification of the corresponding region of the *E. coli* MG1655 chromosome using oligonucleotides P9 and P10 (SEQ ID NOS: 64 and 65) as primers (these primers contained the subsidiary recognition sites for the *Xba*I and *BamH*I endonucleases);
   - the pML-Tc-ter_*thrL* plasmid was obtained by digesting the pML-ter_*thrL* plasmid with the *Kpn*I and *Xba*I restriction endonucleases followed by treatment with Klenow fragment of DNA polymerase I and ligation with the small *EcoR*I-*Van91*I fragment (1317 bp) of pBR322 bearing the tetracycline resistance gene (pBR322 was digested with *EcoR*I and *Van91*I restriction endonucleases and then treated with Klenow fragment of DNA polymerase I).

### Example 1

### (1) Cloning of d0191 gene from P. ananatis SC17 strain

By PCR using the genomic DNA of *P. ananatis* SC17 strain (U.S. Patent No. 6,596,517) as a template, primers *d0191* (Pa)-FW (CGCGGATCCAAGCTTTTCATTATCCAGCAGAGCG, SEQ ID NO: 16), and *d0191*(Pa)-RV (CGCGGATCCTAATGCTGTAGGGCCTGAACCAG, SEQ ID NO: 17), *a d0191* gene fragment containing 300 bp upstream and 200 bp downstream from the *d0191* gene was obtained. Restriction enzyme *Bam*HI sites were designed at the 5' ends of these primers. For PCR, Pyrobest polymerase (Takara) was used, and after a reaction at 94°C for 5 minutes, a cycle of 98°C for 5 seconds, 55°C for 5 seconds and 72°C for 1 minute and 30 seconds was repeated 30 times in the standard reaction composition described in the protocol of the polymerase to amplify the target fragment of about 1.6 kb. The obtained fragment was treated with *Bam*HI and inserted into pSTV29 (Takara) at the *Bam*HI site (inserted in the same direction as the direction of the *lacZ* gene on the vector) to obtain a plasmid pSTV-d0191F (having a chloramphenicol resistance marker) in which *d0191* was cloned. The same PCR fragment was also inserted into pACYC177 (Nippon Gene) at the *Bam*HI site (inserted in the same direction as the direction of the kanamycin resistance gene on the vector) to obtain a plasmid pACYC-d0191F (having kanamycin resistant marker). It was confirmed by sequencing that there was no PCR error. In this way, the plasmids having two kinds of different antibiotic resistance markers and cloned with the same *d0191* region (both had the same p15A origin) were prepared. Furthermore, pHSG-d0191F was prepared, which corresponded to the high copy vector pHSG299 (Takara) inserted with the same DNA fragment of about 1.6 kb as mentioned above at the *BamHI* site (having kanamycin resistant marker, inserted in the same direction as that of the *lacZ* gene on the vector).

### (2) Construction of d0191-enhanced strains from P. ananatis SC 17 strain (SC 17/pSTV-d0191F, SC 17/pHSG-d0191F)

*P. ananatis* SC 17 was transformed with the plasmid pSTV-d0191F constructed as described above to prepare a *d0191*-enhanced strain, SC 17/pSTV-d0191 F strain. As a control strain, a strain introduced with the empty vector, SC17/pSTV29 strain, was also prepared. Furthermore, a *d0191*-enhanced strain SC 17/pHSG-d0191 F strain was also prepared by transforming *P. ananatis* SC17 with the high copy pHSG-d0191 F. As a control strain, a strain introduced with the empty vector, SC 17/pHSG299 strain, was also prepared. The transformation of *P. ananatis* was performed by a conventional method based on electroporation, and selection of the transformants was performed on the LB agar medium (5 g/L of yeast extract, 10 g/L of tryptone, 10 g/L of sodium chloride, 15 g/L of agar) containing an antibiotic corresponding to the antibiotic resistance marker of the plasmid (25 mg/L in the case of chloramphenicol, 20 mg/L in the case of kanamycin).

### (3) Construction of d0191-enhanced strain from E. coli MG1655 strain (MG1655/pSTV-d0191F)

*E. coli* MG1655 was transformed with the plasmid pSTV-d0191F constructed as described above to prepare a *d0191*-enhanced strain, MG1655/pSTV-d0191F strain. As a control strain, a strain introduced with the empty vector, MG1655/pSTV29 strain, was also prepared. The transformation of *E. coli* was performed by a conventional method based on electroporation, and selection of the transformants was performed on the LB agar medium (5 g/L of yeast extract, 10 g/L of tryptone, 10 g/L of sodium chloride, 15 g/L of agar) containing an antibiotic corresponding to the antibiotic resistance marker of the plasmid (25 mg/L in the case of chloramphenicol, 20 mg/L in the case of kanamycin).

### (4) Construction of d0191-deficient strains from P. ananatis SC 17 strain (SC17 d0191::Km^{r} strain, SC17 Dd0191 strain)

Deletion of the *d0191* gene was performed by the method called "Red-driven integration", first developed by Datsenko and Wanner (Proc. Natl. Acad. Sci. USA, 2000, vol. 97, No. 12, pp.6640-6645). According to the "Red-driven integration" method, using a PCR product obtained by using synthetic oligonucleotides in which a part of a target gene is designed on the 5' side, and a part of antibiotic resistance gene is designed on the 3' side, respectively, as primers, a gene-disrupted strain can be constructed in one step.

A DNA fragment comprising homologous sequences of the both ends of the *d0191* gene and an antibiotic resistance gene (kanamycin resistance gene) between them was obtained by PCR. As primers, Dd0191-FW
(CCGTGTCTGAAGCCTATTTTGCCCGCCTGCTGGGCTTGCCTTTTATTGCCTGA AGCCTGCTTTTTTATACTAAGTTGGCA, SEQ ID NO: 18), and Dd0191-RV (CTAGCCCAGTTCGCGCTGCCAGGGCGTAATATCGCCAATGTGCTCGGCAACG CTCAAGTTAGTATAAAAAAGCTGAACGA, SEQ ID NO: 19) were used, and as a template, pME118-(λattL-Km^{r}-λattR) (WO2006/093322A2, SEQ ID NO: 18) was used.

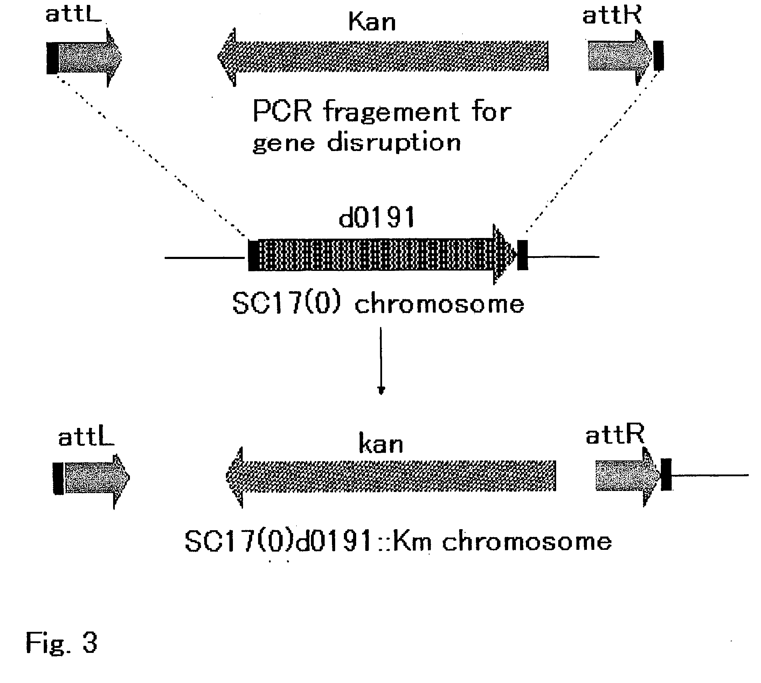

For PCR, Pyrobest polymerase (Takara) was used, and after a reaction at 94°C for 5 minutes, a cycle of 98°C for 5 seconds, 55°C for 5 seconds and 72°C for 2 minute and 30 seconds was repeated 30 times in the standard reaction composition described in the protocol of the polymerase to amplify the target DNA fragment. The obtained DNA fragment (DNA fragment comprising the kanamycin resistant marker and homologous sequences *of d0191* on both sides of the marker, Fig. 3) was introduced into *P. ananatis* SC 17(0)/RSF-Red-TER strain by electroporation to obtain a kanamycin-resistant strain. It was confirmed that the obtained kanamycin-resistant strain was a *d0191* gene-disrupted strain in which the kanamycin resistant cassette was inserted into the *d0191* gene on the chromosome with the homologous sequences of the *d0191* gene region existing at the both ends of the DNA fragment (SC 17(0) d0191::Km^{r} strain, Fig. 3).

Then, by introducing the chromosomal DNA prepared from this SC 17(0) d0191::Km^{r} strain into the SC17 strain by electroporation, a SC17 d0191::Km^{r} strain, which was a *d0191* gene-disrupted strain derived from the SC 17 strain, was finally obtained.

### (5) Construction of plasmid carrying inhibition-desensitized type SAT (serine acetyltransferase) gene (pMIV-CysE5)

It is known that the pMIV-CysE5 plasmid carries the *cysE5* gene encoding a mutant SAT which is desensitized to feedback inhibition (U.S. Patent Published Application No. 20050112731 (A1)). A cysteine-producing bacterium which produces a marked amount of cysteine can be prepared by introducing this plasmid into the bacterium (U.S. Patent Published Application No. 20050112731(A1), U.S. Patent No. 5,972,663 etc.). The construction method of pMIV-CysES is described below.

The mutant allele *E. coli cysE* gene, *cysER5* (U.S. Patent Published Application No. 20050112731(A1)) was obtained by PCR using primers cysEplF (5'-agc-tga-gtc-gac-atg-tcg-tgt-gaa-gaa-ctg-gaa-3', SEQ ID NO: 20), cysER (5'-agc-tga-tct-aga-ata-gat-gat-tac-atc-gca-tcc-3', SEQ ID NO: 21) and the template pMW-PompC-*cysE5* (EP1650296A1). A cycle of 94°C for 0.5 minute, 57°C for 0.5 minute was conducted, then cycles of and 72°C for 1 minute were repeated 27 times, and then the reaction was maintained at 72°C for 7 minutes. The cysEplF primer was designed so as to bind with the start codon ATG of the *E. coli cysE* gene and a downstream sequence, and has a 6-mer *Sal*I site at the 5' end. The cysER primer was designed so as to bind with the stop codon of the *E. coli cysE* gene and an upstream sequence, and has a 6-mer *Xba*I site at the 5' end. A DNA fragment of about 0.7 kb obtained by PCR was digested with *Sal*I and *Xba*I*,* the digestion product was inserted into the pMIV-PompC plasmid which had been similarly digested with *Sal*I and *Xba*I to construct pMIV-CysE5.

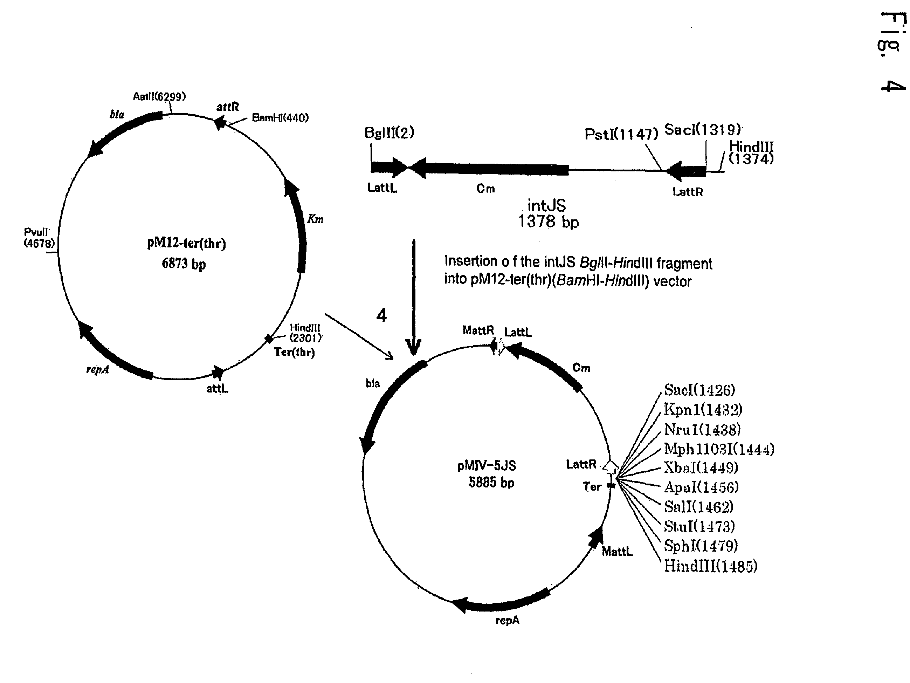

The pMIV-PompC plasmid described above was constructed as follows. By conducting PCR using the genomic DNA of the *E. coli* MG1655 strain as the template, primers PrOMPCF (5'-agc-tga-gtc-gac-aac-cct-ctg-tta-tat-gcc-ttt-a-3', SEQ ID NO: 22) and PrOMPCR (5'-agc-tga-gca-tgc-gag-tga-agg-ttt-tgt-ttt-gac-3', SEQ ID NO: 23), a DNA fragment containing about 0.3 kb of a promoter region of the *ompC* gene was obtained, and this fragment was inserted into the pMIV-5JS plasmid at the *Pae*I and *Sal*I sites to construct pMIV-PompC. The plasmid pMIV-5JS was constructed by ligating the *Bam*HI and *Hin*dIII sites designed beforehand at both ends of the intJS cassette (described later) with the *Bgl*II and *Hin*dIII sites of pM 12-ter(thr) (described later) (Fig. 4).

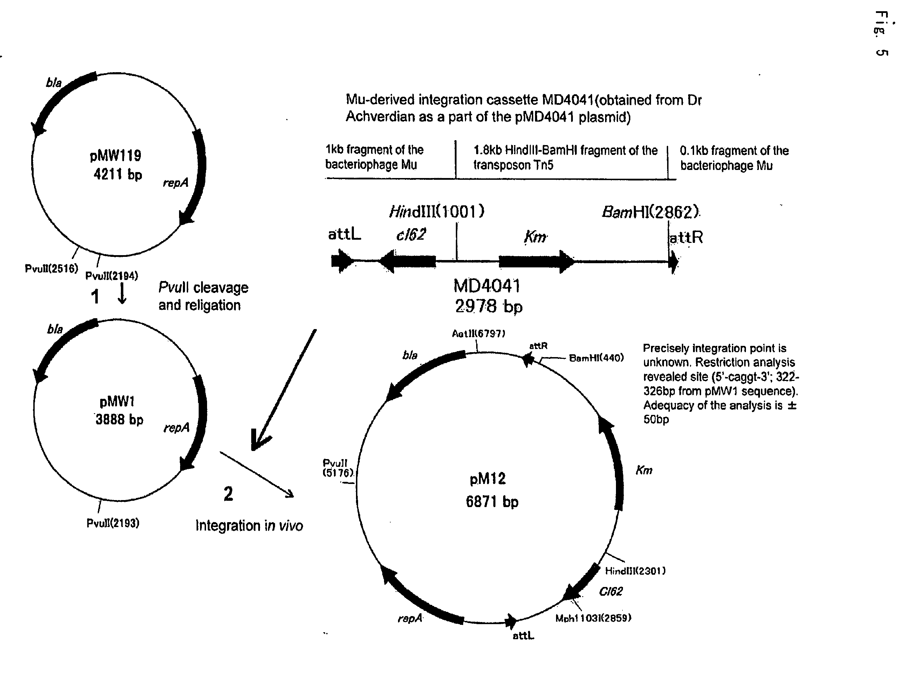

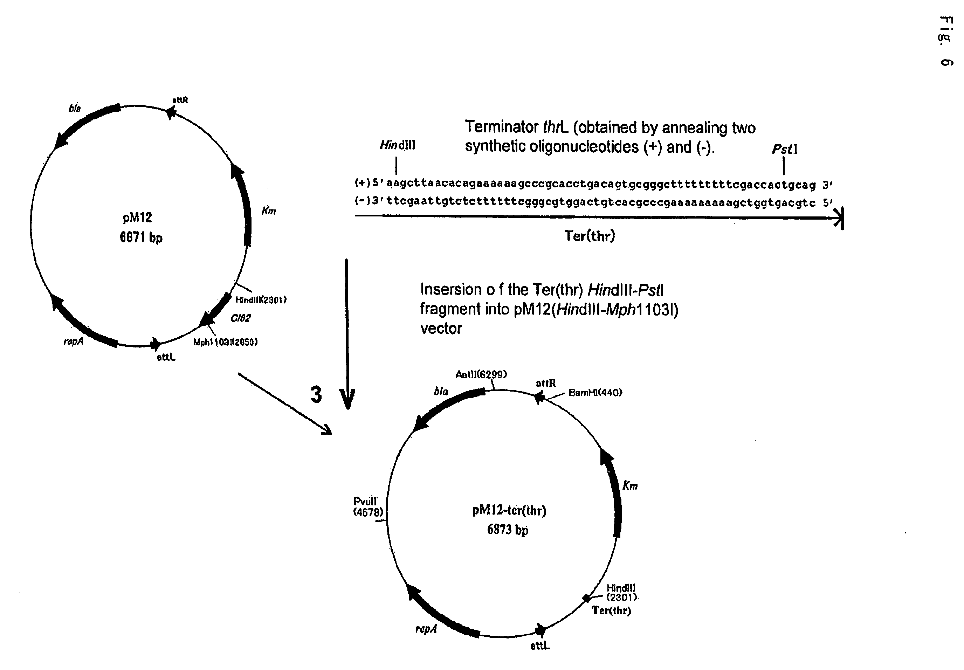

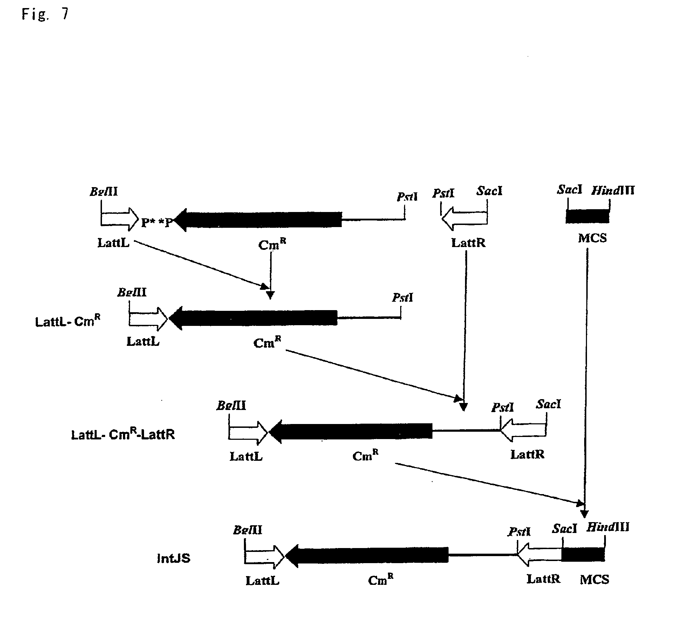

The pM 12-ter(thr) plasmid was constructed (Fig. 6) by inserting a double stranded DNA fragment (thrL terminator, designed to have *Hin*dIII and *Pst*I sites at both ends) produced by annealing a synthetic oligonucleotide (aagcttaaca cagaaaaaag cccgcacctg acagtgcggg cttttttttt cgaccactgc ag, SEQ ID NO: 24) and a complementary synthetic oligonucleotide (ttcgaattgt gtcttttttc gggcgtggac tgtcacgccc gaaaaaaaaa gctggtgacg tc, SEQ ID NO: 25) into the pM12 plasmid which contains the integration cassette derived from Mu phage (EP1486570(A1), Fig. 5) at the *Hin*dIII and Mph1103I sites. The IntJS cassette was constructed by the following procedures (a) to (g) (Fig. 7).
(a) By conducting PCR using an upstream primer (ccagatcttg aagcctgctt ttttatacta agttggc, SEQ ID NO: 26, designed to have a *Bgl*II site), a downstream primer (gaaatcaaat aatgatttta ttttg, SEQ ID NO: 27, phosphorylated) and the pMW118-attL-tet-attR-ter_rrnB plasmid (W02005/010175) as the template, a LattL fragment of 0.12 kbp was obtained.
(b) By conducting PCR using an upstream primer (ttacgccccg ccctgccact catcgc, SEQ ID NO: 28, phosphorylated), a downstream primer (gtcactgcag ctgatgtccg gcggtgcttt tgcc, SEQ ID NO: 29, designed to have a *Pst*I site) and the pACYC184 plasmid (New England Biolabs) as the template, a Cm^{R} fragment of 1.03 kbp was obtained.
(c) By conducting PCR using an upstream primer (cagctgcagt ctgttacagg tcactaatac c, SEQ ID NO: 30, designed to have a *Pst*I site), a downstream primer (ccgagctccg ctcaagttag tataaaaaag ctgaacg, SEQ ID NO: 31, designed to have *SacI* site), and the pMW 18-attL-tet-attR-ter_rrnB plasmid (W02005/010175) as the template, a LattR fragment of 0.16 kbp was obtained.
(d) By ligation of the LattL and Cm^{R} fragments, a LattL-Cm^{R} fragment of 1.15 kbp was obtained.
(e) By ligation of the LattL-Cm^{R} and LattR fragments digested with *Pst*I*,* a LattL-CmR-LattR fragment of 1.31 kbp was obtained.
(f) By annealing a synthetic oligonucleotide (cccgagctcg gtacctcgcg aatgcatcta gatgggcccg tcgactgcag aggcctgcat gcaagcttcc, SEQ ID NO: 32) and with its complementary strand, a double stranded DNA fragment of 70 bp containing a multi-cloning site (MCS) was obtained.
(g) By ligation of the LattL-Cm^{R}-LattR fragment and the double stranded DNA fragment containing a multi-cloning site (MCS), and digested both with *Sac*I*,* an IntJS fragment of 1.38 kbp was obtained.

### (6) Construction of a P. ananatis strain having cysteine-producing ability and enhanced d0191 (SC17/pMIV-CysE5/pACYC-d0191F strain)

The pACYC-d0191F plasmid containing the *d0191* gene (kanamycin resistant) constructed as described above was introduced into the SC17 strain (SC17/pACYC-d0191F), along with the inhibition-desensitized type SAT gene-carrying plasmid pMIV-CysE5 (chloramphenicol resistant) to construct a *P. ananatis* strain having cysteine-producing ability and enhanced *d0191* (SC 17/pMIV-CysE5/pACYC-d0191F strain). Furthermore, as a control strain, SC I 7/pMIV-CysE5/pACYC 177 strain was prepared, which was transformed with the empty vector pACYC177 instead of pACYC-d0191 F.

### (7) Construction of a P. ananatis strain with cysteine-producing ability and deficient in d0191 (SC17 d0191::Km^{r}/pMIV-CysE5 strain).

The *d0191*-deficient strain (SC 17 d0191::Km^{r}), constructed as described above was transformed with the pMIV-CysE5 plasmid to prepare a *d0191*-deficient cysteine-producing bacterium, SC17 d0191::Km^{r}/pMIV-CysE5. The SC17/pMIV-CysE5 strain was prepared as a control, which corresponded to the SC 17 strain transformed with pMIV-CysE5.

### (8) Effect of deletion and enhancement of d0191 on cysteine resistance in the P. ananatis SC 17 strain

In order to investigate the influence of the *d0191* gene on cysteine resistance, the *d0191*-deficient strain SC17 d0191::KM^{r}, the *d0191*-amplified strain SC17/pSTV-d0191F, as well as their control strains (SC 17 and SC17/pSTV29) were each cultured in M9 minimal medium (Sambrook et al., Molecular Cloning, 3rd edition, 2001, Cold Spring Harbor Laboratory Press) which contained cysteine at different concentrations. The difference in cysteine resistance was evaluated by determining change in of the growth. As the resistance to cysteine increased, the OD of the medium more quickly increased, and as the resistance to cysteine decreased, the OD of the mediumincreased more slowly. The procedure of the experiment was as follows. Each strain was precultured overnight in M9 minimal medium containing 0.4% of glucose, but no cysteine (3-ml test tube, 34°C, shaking culture), and then inoculated into the main culture medium. At the time of the inoculation, the OD of the preculture was measured, and the amount inoculated was adjusted so that the main culture begun with the same amount. The OD at the time of the start of the main culture was about 0.007.

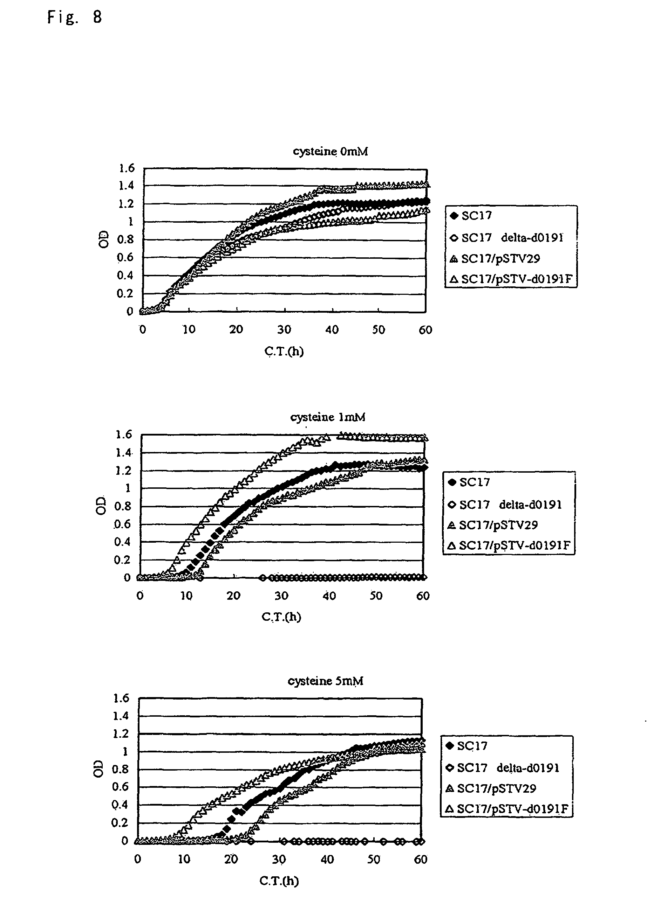

The main culture was performed in 4 ml of M9 minimal medium containing cysteine at the following concentrations: 0 mM, 1 mM, and 5 mM, and 0.4% of glucose using an automatically OD measuring culture apparatus, BIO-PHOTORECORDER TN-1506 (ADVANTEC) and the L-shaped test tube for the apparatus. To the medium for the strains harboring the plasmid, 25 mg/L of chloramphenicol was added. The progress of the culture (growth curves) is shown in Fig. 8. It was observed that as the concentration of cysteine increased, the OD increased more slowly. However, the *d0191*-enhanced strain, SC17/pSTV-d0191F grew more quickly as compared to the control SC17/pSTV29 strain, and it was found to be resistant to cysteine. Furthermore, since the growth of the *d0191*-deficient strain, SC17 *d0191*::Km^{r} was completely inhibited by addition of cysteine, it was found that the cysteine resistance thereof had decreased.

### (9) Effect of enhancing d0191 on cysteine production in E. coli MG1655 strain

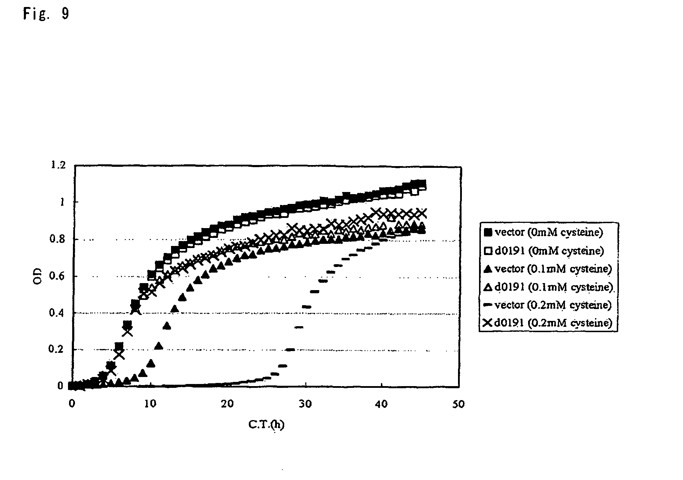

In order to investigate the effect of enhancing the *d0191* gene on cysteine resistance, the cysteine-producing abilities of the *d0191*-amplified strain MG1655/pSTV-d0191F, the control strain thereof MG1655/pSTV29, were each cultured in the M9 minimal medium containing cysteine of different concentrations, and difference in cysteine resistance was evaluated by determining rising of the growth. The culture was performed basically according to the method described in (8), provided that the method was different from the method of (8) in that OD at the start of the main culture was about 0.006, the culture temperature was 37°C, and the cysteine concentrations were 0 mM, 0.1 mM, 0.2 mM. Progress of the culture (growth curves) are shown in Fig. 9. There was observed a tendency that as the concentration of cysteine increased, the rise of OD became later. However, the *d0191*-enhanced strain, MG1655/pSTV-d0191F strain grew earlier compared with the MG1655/pSTV29 strain as the control, and it was found that it was imparted with cysteine resistance. From the above results, it was found that when *d0191* was enhanced, cysteine resistance was enhanced regardless of whether the host is *P. ananatis* or *E. coli.*

### (10) Detection of cysteine desulfhydrase activity of d0191 product by activity staining

The phenotype concerning cysteine resistance suggested a possibility of involvement of the *d0191* product in cysteine decomposition. Therefore, in order to examine whether the *d0191* gene product had the cysteine desulfhydrase activity (henceforth abbreviated as the "CD activity") known as a cysteine decomposition activity, a *d0191*-deficient strain, a *d0191*-enhanced strain and control strains of them were cultured, proteins in cell extracts of them were separated by native PAGE, and the CD activity of them was detected by activity staining. The methods of native PAGE and activity staining were according to the descriptions ofN. Awano et al. (Effect of cysteine desulfhydrase gene disruption on L-cysteine overproduction in *Escherichia coli,* Appl. Microbiol. Biotechnol., 2003 Aug; 62(2-3):239-43).

First, the four strains, the *d0191*-deficient strain SC 17 *d0191*::Km^{r} strain constructed as described above and the control strain thereof, the SC 17 strain, as well as the *d0191*-enhanced strain SC 17/pHSG-d0191 F strain and the control strain thereof, SC17/pHSG299, were cultured by the method described below, and cell extracts of them were prepared. Overnight culture in LB medium (5 g/L of yeast extract, 10 g/L of tryptone, 10 g/L of sodium chloride) of each strain was inoculated into 50 ml of LB medium contained in a Sakaguchi flask so as to be diluted 100 times, and culture was performed with shaking. After 3 hours, cells in the logarithmic phase were collected from the medium (OD was around 0.5), washed with a washing buffer (10 mM Tris-HCl (pH 8.6), 0.1 mM DTT (dithiothreitol), 0.01 mM PLP (pyridoxal-5'-phosphate)), then suspended in 1 ml of the washing buffer, and disrupted by ultrasonication, and the supernatant (cell extract) was obtained by centrifugation. The culture was performed at 34°C for all the strains, and 20 mg/L of kanamycin was added to the medium for the strains harboring a plasmid. Protein concentration of each cell extract prepared as described above was quantified, and the extract was diluted to a protein concentration of 2.5 mg/ml with the washing buffer, and added 5 x loading buffer (10 mM Tris-HCl (pH 8.6), 30% glycerol, 0.005% BPB (Bromophenol Blue)) to prepare a sample of final concentration of 2 mg/ml.

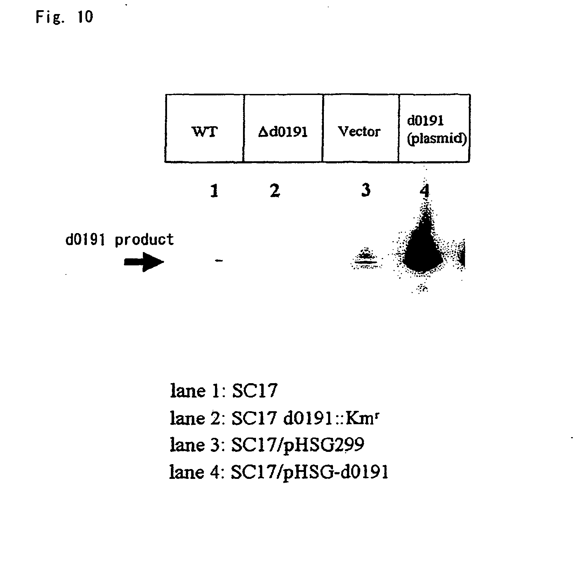

One microliter (2 µg of protein) of each sample was loaded on 10% native PAGE gel (TEFCO), and native PAGE was performed at 4°C and 20 mA for 1.5 hours. The composition of the gel-running buffer used in the native PAGE consisted of 25 mM Tris-HCl (pH 8.3) and 192 mM glycine. After the electrophoresis, the gel was immersed in a staining solution (100 mM Tris-HCl (pH 8.6), 10 mM EDTA (ethylenediaminetetraacetic acid), 50 mM L-cysteine, 0.02 mM PLP, 1.6 mM bismuth(III) chloride), and activity staining was performed at room temperature by slowly shaking the gel until appropriate coloring was observed. The detected bands of the *d0191* product presented by the CD activity are shown in Fig. 10. A band not seen with the *d0191*-deficient strain was observed with the control strain, and increased intensity of this band was observed with the *d0191*-enhanced strain. From these results, it was considered a band of the *d0191* product stained by the CD activity. From the above results, it was revealed that *d0191* coded for a novel cysteine desulfhydrase, and was involved in cysteine resistance.

### (11) Effect of d0191 enhancement on cysteine production in cysteine-producing bacterium

In order to investigate effect of enhancement of the *d0191* gene on cysteine production, cysteine-producing abilities of the cysteine-producing bacterium, *P. ananatis* SC17/pMIV-CysE5/pACYC-d0191F strain, of which *d0191* was enhanced, and the SC17/pMIV-CysE5/pACYC177 strain as a control strain thereof were compared. For the culture, a cysteine production medium (composition: 15 g/L of ammonium sulfate, 1.5 g/L of potassium dihydrogenphosphate, 1 g/L of magnesium sulfate heptahydrate, 0.1 mg/L of thiamine hydrochloride, 1.7 mg/L of ferrous sulfate heptahydrate, 0.15 mg/L of sodium molybdate dihydrate, 0.7 mg/L of cobalt chloride hexahydrate, 1.6 mg/L of manganese chloride tetrahydrate, 0.3 mg/L of zinc sulfate heptahydrate, 0.25 mg/L of copper sulfate pentahydrate, 0.6 g/L of tryptone, 0.3 g/L of yeast extract, 0.6 g/L of sodium chloride, 20 g/L of calcium carbonate, 135 mg/L of L-histidine monohydrochloride monohydrate, 4 g/L of sodium thiosulfate, 2 mg/L of pyridoxine hydrochloride, 60 g/L of glucose, 25 mg/L of chloramphenicol and 20 mg/L of kanamycin) was used.

The culture was performed according to the following procedures. The SC 17/pMIV-CysE5/pACYC-d0191F and the SC 17/pMIV-CysE5/pACYC 177 strains were each applied and spread on LB agar medium containing chloramphenicol and kanamycin, and precultured overnight at 34°C. The cellson about 7 cm on the plate were scraped with an inoculating loop of 10 µl (NUNC Blue Loop), and inoculated into 2 ml of the cysteine production medium in a large test tube (internal diameter: 23 mm, length: 20 cm). The amounts of the inoculated cells were adjusted so that the cell amounts at the time of the start of the culture are substantially the same. The culture was performed at 32°C with shaking. For the both strains, when glucose was completely consumed, the culture was ended (about 22 to 25 hours), and the amount of cysteine which had accumulated in the medium was quantified. The quantification of cysteine was performed by the method described by Gaitonde, M.K. (Biochem. J., 1967 Aug., 104(2):627-33). The experiment was performed in tetraplicate for the both strains, and averages and standard deviations of the accumulated cysteine amounts are shown in Table 1. As shown in Table 1, it was revealed that enhancing *d0191* caused decrease in the accumulation of cysteine.

**Table 1**

| Strain | Gene type | Cys (mg/L) |
|---|---|---|
| SC 17/pMIV-CysE5/pACYC 177 | Vector | 169.5 ± 3.7 |
| SC 17/pMIV-CysE5/pACYC-d0191 F | *d0191* (plasmid) | 71.3 ± 4.6 |

### (9) Effect of a deficiency of d0191 on cysteine-producing ability in cysteine-producing bacterium

Since it became clear that *d0191* participated in decomposition of cysteine, and enhancement of the gene reduced cysteine production, whether suppression of the activity of *d0191* (specifically, deficiency of the gene) had a positive effect on the cysteine production was then examined. In order to examine whether the *d0191-*deficient cysteine-producing strain SC17 d0191::Km^{r}/pMIV-CysE5, prepared as described above had superior cysteine production ability as compared to the *d0191*-non-disrupted strain SC17/pMIV-CysE5, they were cultured for cysteine production, and the abilities thereof were compared. The methods for the cysteine production culture and quantification of cysteine were the same as those described in (11), except that, as for the antibiotics, only chloramphenicol was added to the medium. The experiment was performed in tetraplicate for the control strain, and in octaplicate for the *d0191*-deficient strain. Averages and standard deviations of the accumulated cysteine amounts are shown in Table 2. As shown in Table 2, it was revealed that suppression of the activity of *d0191* had an effect of increasing accumulation amount of cysteine.

**Table 2**

| Strain | Gene type | Cys (mg/L) |
|---|---|---|
| SC17/pMIV-CysE5 | Vector | 202 ± 14 |
| SC17d0191::Km^{r}/pMIV-CysE5 | Δd0191 | 516 ± 46 |

### [Explanation of Sequence Listing]

SEQ ID NO: 1: Nucleotide sequence of *P. ananatis d0191* gene
SEQ ID NO: 2: Amino acid sequence of *P. ananatis* D0191
SEQ ID NO: 3: Nucleotide sequence of *P. ananatis hisD* gene
SEQ ID NOS: 4 to 32: PCR primers
SEQ ID NO: 33: Nucleotide sequence of *Citrobacter koseri d0191* gene
SEQ ID NO: 34: Amino acid sequence of *Citrobacter koseri* D0191
SEQ ID NO: 35: Nucleotide sequence of *Klebsiella pneumoniae d0191* gene
SEQ ID NO: 36: Amino acid sequence of *Klebsiella pneumoniae* D0191
SEQ ID NO: 37: Nucleotide sequence of *Enterobacter* sp. 638 *d0191* gene
SEQ ID NO: 38: Amino acid sequence of *Enterobacter* sp. 638 D0191
SEQ ID NO: 39: Nucleotide sequence of *Salmonella typhimurium d0191* gene
SEQ ID NO: 40: Amino acid sequence of *Salmonella typhimurium* D0191
SEQ ID NO: 41: Nucleotide sequence of *Serratia proteamaculans d0191* gene
SEQ ID NO: 42: Amino acid sequence of *Serratia proteamaculans* D0191
SEQ ID NO: 43: Nucleotide sequence of *Erwinia carotovora d0191* gene
SEQ ID NO: 44: Amino acid sequence *of Erwinia carotovora* D0191
SEQ ID NO: 45: Nucleotide sequence of *Vibrio cholerae d0191* gene
SEQ ID NO: 46: Amino acid sequence of *ribrio cholerae* D0191
SEQ ID NO: 47: Nucleotide sequence of *Pseudomonas fluorescens d0191* gene
SEQ ID NO: 48: Amino acid sequence of *Pseudomonasfluorescens* D0191
SEQ ID NO: 49: Nucleotide sequence of *Streptomyces coelicolor d0191* gene
SEQ ID NO: 50: Amino acid sequence of *Streptomyces coelicolor* D0191
SEQ ID NO: 51: Nucleotide sequence of *Mycobacterium avium d0191* gene
SEQ ID NO: 52: Amino acid sequence of *Mycobacterium avium* D0191
SEQ ID NO: 53: Primer for *attL* amplification
SEQ ID NO: 54: Primer for *attL* amplification
SEQ ID NO: 55: Nucleotide sequence of *attL*
SEQ ID NO: 56: Primer for *attR* amplification
SEQ ID NO: 57: Primer for *attR* amplification
SEQ ID NO: 58: Nucleotide sequence of *attR*
SEQ ID NO: 59: Primer for amplification of DNA fragment containing *bla* gene
SEQ ID NO: 60: Primer for amplification of DNA fragment containing *bla* gene
SEQ ID NO: 61: Primer for amplification of DNA fragment containing *ter_rrnB*
SEQ ID NO: 62: Primer for amplification of DNA fragment containing *ter_rrnB*
SEQ ID NO: 63: Nucleotide sequence of the DNA fragment containing *ter_thrL* terminator
SEQ ID NO: 64: Primer for amplification of DNA fragment containing *ter_thrL* terminator
SEQ ID NO: 65: Primer for amplification of DNA fragment containing *ter_thrL* terminator
SEQ ID NO: 66: Nucleotide sequence of *P. ananatis ybaO* gene
SEQ ID NO: 67: Amino acid sequence of *P. ananatis* YbaO

## Claims

1. A bacterium belonging to the family *Enterobacteriaceae,* which has L-cysteine-producing ability and has been modified to decrease the activity of the protein selected from the group consisting of
(A) a protein comprising the amino acid sequence of SEQ ID NO: 2, and
(B) a protein comprising the amino acid sequence of SEQ ID NO: 2, but which includes substitutions, deletions, insertions or additions of one to 50 amino acid residues, and having cysteine desulfhydrase activity.

2. The bacterium according to claim 1, wherein the activity of the protein is decreased by decreasing expression of a gene encoding the protein or by disrupting the gene.

3. The bacterium according to claim 2, wherein the gene is selected from the group consisting of:
(a) a DNA comprising the nucleotide sequence of SEQ ID NO: 1, and
(b) a DNA which is able to hybridize with a sequence complementary to the nucleotide sequence of SEQ ID NO: 1 or a probe prepared from the nucleotide sequence under stringent conditions, and encodes a protein having cysteine desulfhydrase activity.

4. The bacterium according to any one of claims 1 to 3, in which serine acetyltransferase has been mutated so that feedback inhibition by L-cysteine is attenuated.

5. The bacterium according to any one of claims 1 to 4, which is a *Pantoea* bacterium.

6. The bacterium according to claim 5, which is *Pantoea ananatis.*

7. A method for producing L-cysteine, L-cystine, a derivative thereof or a mixture thereof, which comprises culturing the bacterium according to any one of claims 1 to 6 in a medium and collecting L-cysteine, L-cystine, a derivative thereof or a mixture thereof from the medium.

8. The method according to claim 7, wherein the derivative of L-cysteine is a thiazolidine derivative.

9. A DNA encoding the protein selected from the group consisting of:
(A) a protein comprising the amino acid sequence of SEQ ID NO: 2, and
(B) a protein comprising the amino acid sequence of SEQ ID NO: 2, but which includes substitutions, deletions, insertions or additions of 1 to 50 amino acid residues, and having cysteine desulfhydrase activity.

10. The DNA according to claim 9, which is a DNA selected from the group consisting of:
(a) a DNA comprising the nucleotide sequence of SEQ ID NO: 1, and
(b) a DNA which is able to hybridize with a sequence complementary to the nucleotide sequence of SEQ ID NO: 1 or a probe prepared from the nucleotide sequence under stringent conditions, and encodes a protein having cysteine desulfhydrase activity.

## Patentansprüche

1. Bakterium der Familie *Enterobacteriaceae,* das die Fähigkeit zur Produktion von L-Cystein aufweist und modifiziert worden ist, so dass die Aktivität des aus der folgenden Gruppe ausgewählten Proteins verringert ist:
(A) ein Protein, das die Aminosäuresequenz der SEQ ID NO. 2 enthält, und
(B) ein Protein, das die Aminosäuresequenz der SEQ ID NO. 2 enthält, das jedoch Substitutionen, Deletionen, Insertionen oder Additionen von einem bis 50 Aminosäureresten umfasst und Cysteindesulfhydrase-Aktivität aufweist.

2. Bakterium nach Anspruch 1, wobei die Aktivität des Proteins verringert wird, indem die Expression eines für das Protein kodierenden Gens herabgesetzt ist oder indem das Gen zerstört ist.

3. Bakterium nach Anspruch 2, wobei das Gen aus der folgenden Gruppe ausgewählt ist:
(a) eine DNA, welche die Nucleotidsequenz der SEQ ID NO. 1 enthält und
(b) eine DNA, die mit einer Sequenz, die zu der Nucleotidsequenz der SEQ ID NO. 1 komplementär ist, oder mit einer aus der Nucleotidsequenz hergestellten Sonde unter stringenten Bedingungen hybridisieren kann und für ein Protein mit Cysteindesulfhydrase-Aktivität kodiert.

4. Bakterium nach einem der Ansprüche 1 bis 3, worin Serinacetyltransferase so mutiert worden ist, dass die Rückkopplungshemmung durch L-Cystein abgeschwächt ist.

5. Bakterium nach einem der Ansprüche 1 bis 4, das ein Pantoea-Bakterium ist.

6. Bakterium nach Anspruch 5, das *Pantoea* ananatis ist.

7. Verfahren zum Herstellen von L-Cystein, L-Cystin, eines Derivats davon oder eines Gemisches davon, welches das Kultivieren des Bakteriums nach einem der Ansprüche 1 bis 6 in einem Medium und das Gewinnen von L-Cystein, L-Cystin, eines Derivats davon oder eines Gemisches davon aus dem Medium umfasst.

8. Verfahren nach Anspruch 7, wobei das Derivat von L-Cystein ein Thiazolidinderivat ist.

9. DNA, die für ein Protein kodiert, das aus der folgenden Gruppe ausgewählt ist:
(A) ein Protein, das die Aminosäuresequenz der SEQ ID NO. 2 enthält, und
(B) ein Protein, das die Aminosäuresequenz der SEQ ID NO. 2 enthält, welches jedoch Substitutionen, Deletionen, Insertionen oder Additionen von 1 bis 50 Aminosäureresten umfasst und welches Cysteindesulfhydraseaktivität aufweist.

10. DNA nach Anspruch 9, die eine DNA aus der folgenden Gruppe ist:
(a) eine DNA, welche die Nucleotidsequenz der SEQ ID NO. 1 enthält, und
(b) eine DNA, die mit einer Sequenz, die zu der Nucleotidsequenz der SEQ ID NO. 1 komplementär ist, oder einer aus der Nucleotidsequenz hergestellten Sonde unter stringenten Bedingungen hybridisieren kann und für ein Protein mit Cysteindesulfhydraseaktivität kodiert.

## Revendications

1. Bactérie appartenant à la famille des *Enterobacteriaceae,* laquelle a une capacité de production de L-cystéine et a été modifiée pour diminuer l'activité de la protéine sélectionnée dans le groupe constitué de :
(A) une protéine comprenant la séquence d'acides aminés de SEQ ID NO:2 et
(B) une protéine comprenant la séquence d'acides aminés de SEQ ID NO:2 mais qui comporte des substitutions, des délétions, des insertions ou des additions de 1 à 50 résidus acides aminés et ayant une activité cystéine désulfhydrase.

2. Bactérie selon la revendication 1, où l'activité de la protéine est diminuée par atténuation de l'expression d'un gène codant la protéine ou par rupture du gène.

3. Bactérie selon la revendication 2, où le gène est sélectionné dans le groupe constitué de :
(a) un ADN comprenant la séquence nucléotidique de SEQ ID NO:1 et
(b) un ADN capable de s'hybrider à une séquence complémentaire de la séquence nucléotidique de SEQ ID NO:1 ou à une sonde préparée à partir de la séquence nucléotidique, dans des conditions stringentes, et code une protéine ayant une activité cystéine désulfhydrase.

4. Bactérie selon l'une quelconque des revendications 1 à 3, dans laquelle la sérine acétyltransférase a été mutée de telle sorte que la rétro-inhibition par la L-cystéine est atténuée.

5. Bactérie selon l'une quelconque des revendications 1 à 4, laquelle est une bactérie *Pantoea.*

6. Bactérie selon la revendication 5, laquelle est *Pantoea ananatis*

7. Procédé de production de L-cystéine, de L-cystine, d'un dérivé de celles-ci ou d'un mélange de celles-ci, lequel comprend la culture de la bactérie selon l'une quelconque des revendications 1 à 6 dans un milieu et le recueil de la L-cystéine, de la L-cystine, du dérivé de celles-ci ou du mélange de celles-ci dans le milieu.

8. Procédé selon la revendication 7, où le dérivé de L-cystéine est un dérivé thiazolidine.

9. ADN codant la protéine sélectionnée dans le groupe constitué de :
(A) une protéine comprenant la séquence d'acides aminés de SEQ ID NO:2 et
(B) une protéine comprenant la séquence d'acides aminés de SEQ ID NO:2 mais qui comporte des substitutions, des délétions, des insertions ou des additions de 1 à 50 résidus acides aminés et ayant une activité cystéine désulfhydrase.

10. ADN selon la revendication 9, qui est un ADN sélectionné dans le groupe constitué de :
(a) un ADN comprenant la séquence nucléotidique de SEQ ID NO:1 et
(b) un ADN capable de s'hybrider à une séquence complémentaire de la séquence nucléotidique de SEQ ID NO:1 ou à une sonde préparée à partir de la séquence nucléotidique, dans des conditions stringentes, et code une protéine ayant une activité cystéine désulfhydrase.


### SEQUENCE LISTING

<110> Ajinomoto Co., Inc.
<120> A L-cysteine-producing bacterium and a method for producing L-cysteine
<130> EPA-64697
<150> JP2008-056371
   <151> 2008-03-06
<160> 67
<170> PatentIn version 3.5
<210> 1
   <211> 1035
   <212> DNA
   <213> Pantoea ananatis
<220>
   <221> CDS
   <222> (1)..(1035)
<400> 1
<210> 2
   <211> 344
   <212> PRT
   <213> Pantoea ananatis
<400> 2
<210> 3
   <211> 1308
   <212> DNA
   <213> Pantoea ananatis
<400> 3
   atgagcagaa tcatgacgcc cgtgaactgg gaagcctgca gcagcgaggc gcagcaggcg      60
   ctgttggcac gccctgcgct cgcctcgtct gacagcatca gccagatcgt gcgcgatgtg     120
   ttggtcagag tgaaagagga aggcgatgcg gctttacgag aattcagcgc gcgctttgac     180
   aaggttgaaa cagacgacct gcgcgttacg ccacagcaga tgcaggcggc cagcgatcgc     240
   cttggtgacg agctgaaaca ggcgatggcc gtggccattg gcaatattga aacctttcac     300
   cgtgcgcaga tcctgccgcc ggtggatgtg gaaacgcagc ccggcgtgcg ctgtcagcaa     360
   attacgcgcc cgatgaaatc ggtgggcttg tatattccgg gcggttctgc cccgctgttt     420
   tctaccgttc tgatgctggc taccccggcg cggattgcgg gctgtggtcg cgtggtgctg     480
   tgctcgcccc cgccgattgc tgatgaaatt ctctacgcgg ccaaactttg cggtgtggaa     540
   gaagtgttcc aggtgggtgg atcacaggcg attgccgccc tggcttttgg caccgaaagc     600
   atccctaagg tagataaaat ttttggtccg ggcaacgcgt gggttaccga agccaaacgt     660
   caggtcagcc agcgccttga tggcgcggcg attgatatgc ccgctggccc gtcggaagtg     720
   ctggtgattg ccgatgaagg tgccacaccg gccttcgttg cctctgatct gctgtcgcag     780
   gcggaacacg gccctgactc gcaggtgatt ttactgacgc cttcgctggc gctggccgag     840
   cgcgtcgccg aggcggtgga ggatcagctg gcccagttgc cacgtgcggc gacagcccgc     900
   caggcactgg aaagcagccg cctgatcgtc gcccgggata tgcagcaatg cattgcgatc     960
   tccaaccgct atggtccgga gcacctgatt ctgcaaaccc gcacgccacg ggatctggtg    1020
   gaacagatta ccagcgccgg ttcggttttc ctgggcgact ggtcaccgga atccgcagga    1080
   gattatgctt cgggcaccaa ccacgtgctg ccgacctacg gctataccgc gacatgctcc    1140
   agcctgggcc tggccgactt tcagaaacgc atgacggtac aggagctgac gccgcagggc    1200
   ttcctgaacc tggcggcgac catcgaaacc ctggcggccg ctgaacagct gcacgcccac    1260
   aaaaatgccg tcacgttgcg cgttgccgca ctcaaggagc aagcatga                1308
<210> 4
   <211> 68
   <212> DNA
   <213> Artificial Sequence
<220>
   <223> primer
<400> 4
   ccatagcggt tggagatcgc aatgcattgc tgcatatccc tgaagcctgc ttttttatac      60
   taagttgg                                                              68
<210> 5
   <211> 68
   <212> DNA
   <213> Artificial Sequence
<220>
   <223> primer
<400> 5
   gcccgccagg cactggaaag cagccgcctg atcgtcgccc cgctcaagtt agtataaaaa      60
   agctgaac                                                              68
<210> 6
   <211> 33
   <212> DNA
   <213> Artificial Sequence
<220>
   <223> primer
<400> 6
   tagcgagatc tctgatgtcc ggcggtgctt ttg 33
<210> 7
   <211> 32
   <212> DNA
   <213> Artificial Sequence
<220>
   <223> primer
<400> 7
   aaaaagagct cttacgcccc gccctgccac tc 32
<210> 8
   <211> 34
   <212> DNA
   <213> Artificial Sequence
<220>
   <223> primer
<400> 8
   caggatctag aaggagacat gaacgatgaa catc 34
<210> 9
   <211> 36
   <212> DNA
   <213> Artificial Sequence
<220>
   <223> primer
<400> 9
   gataaggatc cgaaataaaa gaaaatgcca atagga 36
<210> 10
   <211> 31
   <212> DNA
   <213> Artificial Sequence
<220>
   <223> primer
<400> 10
   cctttgagct cgcgggcagt gagcgcaacg c 31
<210> 11
   <211> 48
   <212> DNA
   <213> Artificial Sequence
<220>
   <223> primer
<400> 11
   ctagagcggc cgccgatcgg gatcctcctg tgtgaaattg ttatccgc 48
<210> 12
   <211> 42
   <212> DNA
   <213> Artificial Sequence
<220>
   <223> primer
<400> 12
   ctctacgatc gaggaggtta taaaaaatgg atattaatac tg 42
<210> 13
   <211> 36
   <212> DNA
   <213> Artificial Sequence
<220>
   <223> primer
<400> 13
   tcaaagcggc cgcttcttcg tctgtttcta ctggta 36
<210> 14
   <211> 31
   <212> DNA <213> Artificial Sequence <220>
   <223> primer <400> 14
   cctttggtac cgcgggcagt gagcgcaacg c 31
<210> 15 <211> 34
   <212> DNA
   <213> Artificial Sequence
<220>
   <223> primer
<400> 15
   aacaggaatt ctttgcctgg cggcagtagc gcgg 34
<210> 16
   <211> 34
   <212> DNA
   <213> Artificial Sequence
<220>
   <223> primer
<400> 16
   cgcggatcca agcttttcat tatccagcag agcg 34
<210> 17
   <211> 32
   <212> DNA
   <213> Artificial Sequence
<220>
   <223> primer
<400> 17
   cgcggatcct aatgctgtag ggcctgaacc ag 32
<210> 18
   <211> 80
   <212> DNA
   <213> Artificial Sequence
<220>
   <223> primer
<400> 18
   ccgtgtctga agcctatttt gcccgcctgc tgggcttgcc ttttattgcc tgaagcctgc 60
   ttttttatac taagttggca 80
<210> 19
   <211> 80
   <212> DNA
   <213> Artificial Sequence
<220>
   <223> primer
<400> 19
   ctagcccagt tcgcgctgcc agggcgtaat atcgccaatg tgctcggcaa cgctcaagtt 60
   agtataaaaa agctgaacga 80
<210> 20
   <211> 33
   <212> DNA
   <213> Artificial Sequence
<220>
   <223> primer
<400> 20
   agctgagtcg acatgtcgtg tgaagaactg gaa 33
<210> 21
   <211> 33
   <212> DNA
   <213> Artificial Sequence
<220>
   <223> primer
<400> 21
   agctgatcta gaatagatga ttacatcgca tcc 33
<210> 22
   <211> 34
   <212> DNA
   <213> Artificial Sequence
<220>
   <223> primer
<400> 22
   agctgagtcg acaaccctct gttatatgcc ttta 34
<210> 23

   <211> 33
   <212> DNA
   <213> Artificial Sequence
<220>
   <223> primer
   <400> 23
   agctgagcat gcgagtgaag gttttgtttt gac 33
<210> 24
   <211> 62
   <212> DNA
   <213> Artificial Sequence
<220>
   <223> primer
<400> 24
<210> 25
   <211> 62
   <212> DNA
   <213> Artificial Sequence
<220> <223> primer
<400> 25
   ttcgaattgt gtcttttttc gggcgtggac tgtcacgccc gaaaaaaaaa gctggtgacg 60
   tc 62
<210> 26
   <211> 37
   <212> DNA
   <213> Artificial Sequence
<220>
   <223> primer
<400> 26
   ccagatcttg aagcctgctt ttttatacta agttggc 37
<210> 27
   <211> 25
   <212> DNA
   <213> Artificial Sequence
<220>
   <223> primer
<400> 27
   gaaatcaaat aatgatttta ttttg 25
<210> 28
   <211> 26
   <212> DNA
   <213> Artificial Sequence
<220>
   <223> primer
<400> 28
   ttacgccccg ccctgccact catcgc 26
<210> 29
   <211> 34
   <212> DNA
   <213> Artificial Sequence
<220>
   <223> primer
<400> 29
   gtcactgcag ctgatgtccg gcggtgcttt tgcc 34
<210> 30
   <211> 31
   <212> DNA
   <213> Artificial Sequence
<220>
   <223> primer
<400> 30
   cagctgcagt ctgttacagg tcactaatac c 31
<210> 31
   <211> 37
   <212> DNA
   <213> Artificial Sequence
<220>
   <223> primer
<400> 31
   ccgagctccg ctcaagttag tataaaaaag ctgaacg 37
<210> 32
   <211> 70
   <212> DNA
   <213> Artificial sequence
<220>
   <223> primer
<400> 32
   cccgagctcg gtacctcgcg aatgcatcta gatgggcccg tcgactgcag aggcctgcat 60
   gcaagcttcc 70
<210> 33
   <211> 1056
   <212> DNA
   <213> Citrobacter koseri
<220>
   <221> CDS
   <222> (1)..(1056)
<400> 33
   atg atg aat agc acc tgg gtt aaa aac gcc att aat gaa att aac gct 48
   Met Met Asn Ser Thr Trp Val Lys Asn Ala Ile Asn Glu Ile Asn Ala
   1 5 10 15
   gac tat cag cgt tca gcc gac acg cat ctg atc cgg ttg tcg cta cca 96
   Asp Tyr Gln Arg Ser Ala Asp Thr His Leu Ile Arg Leu Ser Leu Pro
   20 25 30
   gcg ttt ccg ggc atc cag atc tac ctg aag gat gaa agc acc cat ccg 144
   Ala Phe Pro Gly Ile Gln Ile Tyr Leu Lys Asp Glu Ser Thr His Pro
   35 40 45
   acc ggc agc ctg aag cac cgt ctg gcg cgt tca ttg ttc ctg tat ggg 192
   Thr Gly Ser Leu Lys His Arg Leu Ala Arg Ser Leu Phe Leu Tyr Gly
   50 55 60
   ttg tgt aac ggc tgg att aaa gaa aac acg acc att att gaa tcc tcc 240
   Leu Cys Asn Gly Trp Ile Lys Glu Asn Thr Thr Ile Ile Glu Ser Ser
   65 70 75 80
   tcc ggt tca acg gcg gtt tcc gaa gcc tgg ttc gcc cga ctg ctc ggc 288
   Ser Gly Ser Thr Ala Val Ser Glu Ala Trp Phe Ala Arg Leu Leu Gly
   85 90 95
   ctg ccg ttt atc gcg gta atg cct tcc tgc acg gca aaa cgc aaa atc 336
   Leu Pro Phe Ile Ala Val Met Pro Ser Cys Thr Ala Lys Arg Lys Ile
   100 105 110
   gaa caa att gcg ttt tat ggc ggt cgc tgc cac ttt gtc gaa agc gcc 384
   Glu Gln Ile Ala Phe Tyr Gly Gly Arg Cys His Phe Val Glu Ser Ala
   115 120 125
   tgc gag att tac gcc gcc tct gag cag ctg gcg cac gaa ctg aac ggt 432
   Cys Glu Ile Tyr Ala Ala Ser Glu Gln Leu Ala His Glu Leu Asn Gly
   130 135 140
   cac tat atg gat cag ttc acc tat gcc gaa cgc gcc acc gac tgg cgc 480
   His Tyr Met Asp Gln Phe Thr Tyr Ala Glu Arg Ala Thr Asp Trp Arg
   145 150 155 160
   ggc aac aat aat atc gcc gac agt att ttc cat cag atg cag cgt gag 528
   Gly Asn Asn Asn Ile Ala Asp Ser Ile Phe His Gln Met Gln Arg Glu
   165 170 175
   cct aat ccg gtg ccg aag cat atc gtg atg agc gcg ggg acg ggc ggc 576
   Pro Asn Pro Val Pro Lys His Ile Val Met Ser Ala Gly Thr Gly Gly
   180 185 190
   acg tca gcg aca att ggc cgc tat att cgc tgt cat ggg tat gac acg 624
   Thr Ser Ala Thr Ile Gly Arg Tyr Ile Arg Cys His Gly Tyr Asp Thr
   195 200 205
   cgc ctg atg gtg gtt gac ccg gaa aac tcg gtc ttc ctg ccc tac tgg 672
   Arg Leu Met Val Val Asp Pro Glu Asn Ser Val Phe Leu Pro Tyr Trp
   210 215 220
   cag gat cgc gac gcc aca ttg cgc agc ccg gtg ggc agc aaa att gag 720
   Gln Asp Arg Asp Ala Thr Leu Arg Ser Pro Val Gly Ser Lys Ile Glu
   225 230 235 240
   ggg att ggc cgc ccg cgc gtg gaa ccc tcc ttc atc ccg gat gtg gtg 768
   Gly Ile Gly Arg Pro Arg Val Glu Pro Ser Phe Ile Pro Asp Val Val
   245 250 255
   gac gaa atg ctg cgc gta ccc gac gcc gcc agc gtc gcg acc gcg cac 816
   Asp Glu Met Leu Arg Val Pro Asp Ala Ala Ser Val Ala Thr Ala His
   260 265 270
   tgg ctg gaa aca cag ctg ggc cgt aaa gtg ggc gcc tcc acc ggc act 864
   Trp Leu Glu Thr Gln Leu Gly Arg Lys Val Gly Ala Ser Thr Gly Thr
   275 280 285
   aat atg tgg gga acg ttg cag atc gca gcc aga atg cgc gac gcc gga 912
   Asn Met Trp Gly Thr Leu Gln Ile Ala Ala Arg Met Arg Asp Ala Gly
   290 295 300
   gag acc ggt tct ctc gtc acg ctg ctg tgt gac agc ggc gaa cgc tat 960
   Glu Thr Gly Ser Leu Val Thr Leu Leu Cys Asp Ser Gly Glu Arg Tyr
   305 310 315 320
   ctc gac acc tat tac aat ccc cag tgg gtt aac gat cac att ggc gat 1008
   Leu Asp Thr Tyr Tyr Asn Pro Gln Trp Val Asn Asp His Ile Gly Asp
   325 330 335
   tta gcg ccg tgg cag gcg gaa att gcc caa ctg ctg aac gtc cgt taa 1056
   Leu Ala Pro Trp Gln Ala Glu Ile Ala Gln Leu Leu Asn Val Arg
   340 345 350
<210> 34
   <211> 351
   <212> PRT
   <213> Citrobacter koseri
<400> 34
   Met Met Asn Ser Thr Trp Val Lys Asn Ala Ile Asn Glu Ile Asn Ala
   1               5                   10                  15
   Asp Tyr Gln Arg Ser Ala Asp Thr His Leu Ile Arg Leu Ser Leu Pro
               20                  25                  30
   Ala Phe Pro Gly Ile Gln Ile Tyr Leu Lys Asp Glu Ser Thr His Pro
           35                  40                  45
   Thr Gly Ser Leu Lys His Arg Leu Ala Arg Ser Leu Phe Leu Tyr Gly
       50                  55                  60
   Leu Cys Asn Gly Trp Ile Lys Glu Asn Thr Thr Ile Ile Glu Ser Ser
   65                  70                  75                  80
   Ser Gly Ser Thr Ala Val Ser Glu Ala Trp Phe Ala Arg Leu Leu Gly
                   85                  90                  95
   Leu Pro Phe Ile Ala Val Met Pro Ser Cys Thr Ala Lys Arg Lys Ile
               100                 105                 110
   Glu Gln Ile Ala Phe Tyr Gly Gly Arg Cys His Phe Val Glu Ser Ala
           115                 120                 125
   Cys Glu Ile Tyr Ala Ala Ser Glu Gln Leu Ala His Glu Leu Asn Gly
       130                 135                 140
   His Tyr Met Asp Gln Phe Thr Tyr Ala Glu Arg Ala Thr Asp Trp Arg
   145                 150                 155                 160
   Gly Asn Asn Asn Ile Ala Asp Ser Ile Phe His Gln Met Gln Arg Glu
                   165                 170                 175
   Pro Asn Pro Val Pro Lys His Ile Val Met Ser Ala Gly Thr Gly Gly
               180                 185                 190
   Thr Ser Ala Thr Ile Gly Arg Tyr Ile Arg Cys His Gly Tyr Asp Thr
           195                 200                 205
   Arg Leu Met Val Val Asp Pro Glu Asn Ser Val Phe Leu Pro Tyr Trp
       210                 215                 220
   Gln Asp Arg Asp Ala Thr Leu Arg Ser Pro Val Gly Ser Lys Ile Glu
   225                 230                 235                 240
   Gly Ile Gly Arg Pro Arg Val Glu Pro Ser Phe Ile Pro Asp Val Val
                   245                 250                 255
   Asp Glu Met Leu Arg Val Pro Asp Ala Ala Ser Val Ala Thr Ala His
               260                 265                 270
   Trp Leu Glu Thr Gln Leu Gly Arg Lys Val Gly Ala Ser Thr Gly Thr
           275                 280                 285
   Asn Met Trp Gly Thr Leu Gln Ile Ala Ala Arg Met Arg Asp Ala Gly
       290                 295                 300
   Glu Thr Gly Ser Leu Val Thr Leu Leu Cys Asp Ser Gly Glu Arg Tyr
   305                 310                 315                 320
   Leu Asp Thr Tyr Tyr Asn Pro Gln Trp Val Asn Asp His Ile Gly Asp
                   325                 330                 335
   Leu Ala Pro Trp Gln Ala Glu Ile Ala Gln Leu Leu Asn Val Arg
               340                 345                 350
<210> 35
   <211> 1053
   <212> DNA
   <213> Klebsiella pneumoniae
<220>
   <221> CDS
   <222> (1)..(1053)
<400> 35
   atg aat agc gcc tgg gtt aaa cat gcc att agc gaa atc aac gct gac      48
   Met Asn Ser Ala Trp Val Lys His Ala Ile Ser Glu Ile Asn Ala Asp
   1               5                   10                  15
   tat cag cgc tcc gcc gac acg cac ctg atc cgt ctg ccg ctg ccc gct      96
   Tyr Gln Arg Ser Ala Asp Thr His Leu Ile Arg Leu Pro Leu Pro Ala
               20                  25                  30
   ttt ccg ggc atc cac ctg tat ctg aaa gat gaa agc acc cac ccc acc      144
   Phe Pro Gly Ile His Leu Tyr Leu Lys Asp Glu Ser Thr His Pro Thr
           35                  40                  45
   ggc agc ctg aag cat cgc ctg gcg cgg tcg ctg ttt ctc tat ggc ttg      192
   Gly Ser Leu Lys His Arg Leu Ala Arg Ser Leu Phe Leu Tyr Gly Leu
       50                  55                  60
   tgc aac ggc tgg atc aaa gaa ggc acg cca att ata gag tcc tcc tca      240
   Cys Asn Gly Trp Ile Lys Glu Gly Thr Pro Ile Ile Glu Ser Ser Ser
   65                  70                  75                  80
   ggt tca acc gcc gtt tcg gag gcc tat ttc gcc cgc ctg ctg ggg ctg      288
   Gly Ser Thr Ala Val Ser Glu Ala Tyr Phe Ala Arg Leu Leu Gly Leu
                   85                  90                  95
   ccg ttt atc gct gtg atg cct tcc tgc acc gcc aag cgc aaa atc gaa      336
   Pro Phe Ile Ala Val Met Pro Ser Cys Thr Ala Lys Arg Lys Ile Glu
               100                 105                 110
   cag atc gag ttc tac ggc gga cgc tgc cat ttt gtg cag agc gcc ggc      384
   Gln Ile Glu Phe Tyr Gly Gly Arg Cys His Phe Val Gln Ser Ala Gly
           115                 120                 125
   gaa atc tac gcg gct tcc gaa acg ctg gcc cgc gag ctg aac ggc cat      432
   Glu Ile Tyr Ala Ala Ser Glu Thr Leu Ala Arg Glu Leu Asn Gly His
       130                 135                 140
   tat atg gat cag ttt acc ttc gcc gag cgg gcc acc gac tgg cgc ggc      480
   Tyr Met Asp Gln Phe Thr Phe Ala Glu Arg Ala Thr Asp Trp Arg Gly
   145                 150                 155                 160
   aac aac aat atc gcc gac agt att ttc cgc cag atg agc cat gag ccg      528
   Asn Asn Asn Ile Ala Asp Ser Ile Phe Arg Gln Met Ser His Glu Pro
                   165                 170                 175
   cac ccg cag ccg tca tgg atc gtc atg agc gcc ggc acc ggc ggc acc      576
   His Pro Gln Pro Ser Trp Ile Val Met Ser Ala Gly Thr Gly Gly Thr
               180                 185                 190
   tca gcg acc att ggc cgc tat att cgc agc cag ggc tac gag acg cag      624
   Ser Ala Thr Ile Gly Arg Tyr Ile Arg Ser Gln Gly Tyr Glu Thr Gln
           195                 200                 205
   ctg atg gtc gtt gat ccg cag aat tcg gtg ttc ctc gat tac tgg cag      672
   Leu Met Val Val Asp Pro Gln Asn Ser Val Phe Leu Asp Tyr Trp Gln
       210                 215                 220
   acc cgc gac gcc agc ctg cgc agc ccg gtc ggc agc aaa att gaa ggc      720
   Thr Arg Asp Ala Ser Leu Arg Ser Pro Val Gly Ser Lys Ile Glu Gly
   225                 230                 235                 240
   atc gga cgg ccg cgc gtc gag ccg tcg ttt att ccc gac gtt gtc gat      768
   Ile Gly Arg Pro Arg Val Glu Pro Ser Phe Ile Pro Asp Val Val Asp
                   245                 250                 255
   gag atg ctg cgc gtg ccg gat gcg gcg agc gta gcc acc gcc ctg tgg      816
   Glu Met Leu Arg Val Pro Asp Ala Ala Ser Val Ala Thr Ala Leu Trp
               260                 265                 270
   ctg gaa acg cag ctt ggg cgc aag gtc ggc gcc tcg acc ggc acc aat      864
   Leu Glu Thr Gln Leu Gly Arg Lys Val Gly Ala Ser Thr Gly Thr Asn
           275                 280                 285
   atg tgg ggc gta ttg cag ctg gcc acg cgg atg cgc gag gaa ggt cgc      912
   Met Trp Gly Val Leu Gln Leu Ala Thr Arg Met Arg Glu Glu Gly Arg
       290                 295                 300
   acc ggc tcg ata gtg aca ctc ctg tgc gac agc ggc gag cgt tac ctg      960
   Thr Gly Ser Ile Val Thr Leu Leu Cys Asp Ser Gly Glu Arg Tyr Leu
   305                 310                 315                 320
   gaa agc tat tac aac ccg cag tgg gtg gcg gac aat atc ggc gat att      1008
   Glu Ser Tyr Tyr Asn Pro Gln Trp Val Ala Asp Asn Ile Gly Asp Ile
                   325                 330                 335
   gcg ccc tgg cag gcg gag atc gcc ggt ctg gtc gaa agg cga taa          1053
   Ala Pro Trp Gln Ala Glu Ile Ala Gly Leu Val Glu Arg Arg
               340                 345                 350
<210> 36
   <211> 350
   <212> PRT
   <213> Klebsiella pneumoniae
<400> 36
   Met Asn Ser Ala Trp Val Lys His Ala Ile Ser Glu Ile Asn Ala Asp
   1               5                   10                  15
   Tyr Gln Arg Ser Ala Asp Thr His Leu Ile Arg Leu Pro Leu Pro Ala
               20                  25                  30
   Phe Pro Gly Ile His Leu Tyr Leu Lys Asp Glu Ser Thr His Pro Thr
           35                  40                  45
   Gly Ser Leu Lys His Arg Leu Ala Arg Ser Leu Phe Leu Tyr Gly Leu
       50                  55                  60
   Cys Asn Gly Trp Ile Lys Glu Gly Thr Pro Ile Ile Glu Ser Ser Ser
   65                  70                  75                  80
   Gly Ser Thr Ala Val Ser Glu Ala Tyr Phe Ala Arg Leu Leu Gly Leu
                   85                  90                  95
   Pro Phe Ile Ala Val Met Pro Ser Cys Thr Ala Lys Arg Lys Ile Glu
               100                 105                 110
   Gln Ile Glu Phe Tyr Gly Gly Arg Cys His Phe Val Gln Ser Ala Gly
           115                 120                 125
   Glu Ile Tyr Ala Ala Ser Glu Thr Leu Ala Arg Glu Leu Asn Gly His
       130                 135                 140
   Tyr Met Asp Gln Phe Thr Phe Ala Glu Arg Ala Thr Asp Trp Arg Gly
   145                 150                 155                 160
   Asn Asn Asn Ile Ala Asp Ser Ile Phe Arg Gln Met Ser His Glu Pro
                   165                 170                 175
   His Pro Gln Pro Ser Trp Ile Val Met Ser Ala Gly Thr Gly Gly Thr
               180                 185                 190
   Ser Ala Thr Ile Gly Arg Tyr Ile Arg Ser Gln Gly Tyr Glu Thr Gln
           195                 200                 205
   Leu Met Val Val Asp Pro Gln Asn Ser Val Phe Leu Asp Tyr Trp Gln
       210                 215                 220
   Thr Arg Asp Ala Ser Leu Arg Ser Pro Val Gly Ser Lys Ile Glu Gly
   225                 230                 235                 240
   Ile Gly Arg Pro Arg Val Glu Pro Ser Phe Ile Pro Asp Val Val Asp
                   245                 250                 255
   Glu Met Leu Arg Val Pro Asp Ala Ala Ser Val Ala Thr Ala Leu Trp
               260                 265                 270
   Leu Glu Thr Gln Leu Gly Arg Lys Val Gly Ala Ser Thr Gly Thr Asn
           275                 280                 285
   Met Trp Gly Val Leu Gln Leu Ala Thr Arg Met Arg Glu Glu Gly Arg
       290                 295                 300
   Thr Gly Ser Ile Val Thr Leu Leu Cys Asp Ser Gly Glu Arg Tyr Leu
   305                 310                 315                 320
   Glu Ser Tyr Tyr Asn Pro Gln Trp Val Ala Asp Asn Ile Gly Asp Ile
                   325                 330                 335
   Ala Pro Trp Gln Ala Glu Ile Ala Gly Leu Val Glu Arg Arg
               340                 345                 350
<210> 37
   <211> 1047
   <212> DNA
   <213> Enterobacter sp.638
<220>
   <221> CDS
   <222> (1)..(1047)
<400> 37
   atg aat agc tct tgg gtc aaa cat gcg att agc gaa att aat gcc gat 48
   Met Asn Ser Ser Trp Val Lys His Ala Ile Ser Glu Ile Asn Ala Asp
   1 5 10 15
   tat caa cgt tcg gcg gat acg cat ctg att cgc ctc gcg ctg ccg ggt 96
   Tyr Gln Arg Ser Ala Asp Thr His Leu Ile Arg Leu Ala Leu Pro Gly
   20 25 30
   ttt gca ggc att cag ctc tat cta aaa gat gaa agt act cac cct aca 144
   Phe Ala Gly Ile Gln Leu Tyr Leu Lys Asp Glu Ser Thr His Pro Thr
   35 40 45
   ggc agc ctc aag cat cgt ctg gcg cga tcg ttg ttt ttg tat ggt ttg 192
   Gly Ser Leu Lys His Arg Leu Ala Arg Ser Leu Phe Leu Tyr Gly Leu
   50 55 60
   tgc aac ggc tgg atc aaa gaa ggc aca acg atc att gaa tcg tca tcg 240
   Cys Asn Gly Trp Ile Lys Glu Gly Thr Thr Ile Ile Glu Ser Ser Ser
   65 70 75 80
   ggt tca acg gcc gtg tca gaa gcc tat ttc gcg cgc ctg ctt ggc ctg 288
   Gly Ser Thr Ala Val Ser Glu Ala Tyr Phe Ala Arg Leu Leu Gly Leu
   85 90 95
   ccg ttt atc gcc gtg atg ccc tcc tgt acc gcg aaa cgc aaa atc gaa 336
   Pro Phe Ile Ala Val Met Pro Ser Cys Thr Ala Lys Arg Lys Ile Glu
   100 105 110
   cag atc gaa ttc tac ggc ggc cgc tgc cac ttt gtg gaa agc gcc tgc 384
   Gln Ile Glu Phe Tyr Gly Gly Arg Cys His Phe Val Glu Ser Ala Cys
   115 120 125
   gag att tac gcc gcc tcg gaa atg ctc gcc cgt gag ctg aac ggt cac 432
   Glu Ile Tyr Ala Ala Ser Glu Met Leu Ala Arg Glu Leu Asn Gly His
   130 135 140
   tat atg gac cag ttt acg ttt gct gag cgt gcg acc gac tgg cgc ggg 480
   Tyr Met Asp Gln Phe Thr Phe Ala Glu Arg Ala Thr Asp Trp Arg Gly
   145 150 155 160
   aac aac aac att gcg gac agt att ttc cgt cag atg aac aat gag ccg 528
   Asn Asn Asn Ile Ala Asp Ser Ile Phe Arg Gln Met Asn Asn Glu Pro
   165 170 175
   cac ccc cag ccg tcg tat atc gtc atg agc gcc ggg acg ggc ggt acc 576
   His Pro Gln Pro Ser Tyr Ile Val Met Ser Ala Gly Thr Gly Gly Thr
   180 185 190
   tcg gcg acg att ggt cgc tat atc cgc tgc cag ggc tat gac act caa 624
   Ser Ala Thr Ile Gly Arg Tyr Ile Arg Cys Gln Gly Tyr Asp Thr Gln
   195 200 205
   ctc atg gtg gtt gat ccg cag aat tct gtg ttt atg gat tac tgg cag 672
   Leu Met Val Val Asp Pro Gln Asn Ser Val Phe Met Asp Tyr Trp Gln
   210 215 220
   agc cgc gat gcc acg ctt cgc agc cca gtg ggc agt aaa atc gaa ggg 720
   Ser Arg Asp Ala Thr Leu Arg Ser Pro Val Gly Ser Lys Ile Glu Gly
   225 230 235 240
   att ggc cgc ccg cgc gta gag cct tct ttc att cct gat gtc gtc gat 768
   Ile Gly Arg Pro Arg Val Glu Pro Ser Phe Ile Pro Asp Val Val Asp
   245 250 255
   gag atg ttg cgt gtg cca gat gcg gca agc gtg gcg acg gca cac tgg 816
   Glu Met Leu Arg Val Pro Asp Ala Ala Ser Val Ala Thr Ala His Trp
   260 265 270
   ctg gaa acg cag ctt ggc cgc aaa gtg ggc gcg tca acc ggg acc aat 864
   Leu Glu Thr Gln Leu Gly Arg Lys Val Gly Ala Ser Thr Gly Thr Asn
   275 280 285
   atg tgg ggc gtt ctg cac ctg gct tca cga atg cgc gag gaa ggc cgc 912
   Met Trp Gly Val Leu His Leu Ala Ser Arg Met Arg Glu Glu Gly Arg
   290 295 300
   acc ggt tct atc gtt acc ctg ctg tgc gac agc ggc gag cgc tat ctc 960
   Thr Gly Ser Ile Val Thr Leu Leu Cys Asp Ser Gly Glu Arg Tyr Leu
   305 310 315 320
   gac acc tat tac aac gca gag tgg gtg aaa acc aat atc ggg gat atc 1008
   Asp Thr Tyr Tyr Asn Ala Glu Trp Val Lys Thr Asn Ile Gly Asp Ile
   325 330 335
   gaa ccg tgg aaa gcg cag atc gcg cag atg ctg aaa taa 1047
   Glu Pro Trp Lys Ala Gln Ile Ala Gln Met Leu Lys
   340 345
<210> 38
   <211> 348
   <212> PRT
   <213> Enterobacter sp.638
<400> 38
   Met Asn Ser Ser Trp Val Lys His Ala Ile Ser Glu Ile Asn Ala Asp
   1               5                   10                  15
   Tyr Gln Arg Ser Ala Asp Thr His Leu Ile Arg Leu Ala Leu Pro Gly
               20                  25                  30
   Phe Ala Gly Ile Gln Leu Tyr Leu Lys Asp Glu Ser Thr His Pro Thr
           35                  40                  45
   Gly Ser Leu Lys His Arg Leu Ala Arg Ser Leu Phe Leu Tyr Gly Leu
       50                  55                  60
   Cys Asn Gly Trp Ile Lys Glu Gly Thr Thr Ile Ile Glu Ser Ser Ser
   65                  70                  75                  80
   Gly Ser Thr Ala Val Ser Glu Ala Tyr Phe Ala Arg Leu Leu Gly Leu
                   85                  90                  95
   Pro Phe Ile Ala Val Met Pro Ser Cys Thr Ala Lys Arg Lys Ile Glu
               100                 105                 110
   Gln Ile Glu Phe Tyr Gly Gly Arg Cys His Phe Val Glu Ser Ala Cys
           115                 120                 125
   Glu Ile Tyr Ala Ala Ser Glu Met Leu Ala Arg Glu Leu Asn Gly His
       130                 135                 140
   Tyr Met Asp Gln Phe Thr Phe Ala Glu Arg Ala Thr Asp Trp Arg Gly
   145                 150                 155                 160
   Asn Asn Asn Ile Ala Asp Ser Ile Phe Arg Gln Met Asn Asn Glu Pro
                   165                 170                 175
   His Pro Gln Pro Ser Tyr Ile Val Met Ser Ala Gly Thr Gly Gly Thr
               180                 185                 190
   Ser Ala Thr Ile Gly Arg Tyr Ile Arg Cys Gln Gly Tyr Asp Thr Gln
           195                 200                 205
   Leu Met Val Val Asp Pro Gln Asn Ser Val Phe Met Asp Tyr Trp Gln
       210                 215                 220
   Ser Arg Asp Ala Thr Leu Arg Ser Pro Val Gly Ser Lys Ile Glu Gly
   225                 230                 235                 240
   Ile Gly Arg Pro Arg Val Glu Pro Ser Phe Ile Pro Asp Val Val Asp
                   245                 250                 255
   Glu Met Leu Arg Val Pro Asp Ala Ala Ser Val Ala Thr Ala His Trp
               260                 265                 270
   Leu Glu Thr Gln Leu Gly Arg Lys Val Gly Ala Ser Thr Gly Thr Asn
           275                 280                 285
   Met Trp Gly Val Leu His Leu Ala Ser Arg Met Arg Glu Glu Gly Arg
       290                 295                 300
   Thr Gly Ser Ile Val Thr Leu Leu Cys Asp Ser Gly Glu Arg Tyr Leu
   305                 310                 315                 320
   Asp Thr Tyr Tyr Asn Ala Glu Trp Val Lys Thr Asn Ile Gly Asp Ile
                   325                 330                 335
   Glu Pro Trp Lys Ala Gln Ile Ala Gln Met Leu Lys
               340                 345
<210> 39
   <211> 1056
   <212> DNA
   <213> Salmonella typhimurium
<220>
   <221> CDS
   <222> (1)..(1056)
<400> 39
   atg atg agt agc aat tgg gtt aaa aat gcc att aat gaa att aac gct        48
   Met Met Ser Ser Asn Trp Val Lys Asn Ala Ile Asn Glu Ile Asn Ala
   1               5                   10                  15
   gac cac cag cgc tcg gcg gat acg cat ctt att cgt ttg ccc ctg tcg        96
   Asp His Gln Arg Ser Ala Asp Thr His Leu Ile Arg Leu Pro Leu Ser
               20                  25                  30
   gca ttt cca ggt atc cag ctt tac ctg aaa gac gaa agc acg cat ccc       144
   Ala Phe Pro Gly Ile Gln Leu Tyr Leu Lys Asp Glu Ser Thr His Pro
           35                  40                  45
   acc ggt agc ctc aag cat cgt ctg gcg cgc tcg ctg ttc ctt tat ggg       192
   Thr Gly Ser Leu Lys His Arg Leu Ala Arg Ser Leu Phe Leu Tyr Gly
       50                  55                  60
   ttg tgc aat ggc tgg att aaa gaa ggc act ccg att att gaa gcg tca       240
   Leu Cys Asn Gly Trp Ile Lys Glu Gly Thr Pro Ile Ile Glu Ala Ser
   65                  70                  75                  80
   tca ggc tca acg gcg att tcc gaa gcc tgg ttc gcg cgt tta ctg gga       288
   Ser Gly Ser Thr Ala Ile Ser Glu Ala Trp Phe Ala Arg Leu Leu Gly
                   85                  90                  95
   ctg ccg ttc atc gcc gtc atg ccc gcc tgt acc gca aaa cgt aaa atc       336
   Leu Pro Phe Ile Ala Val Met Pro Ala Cys Thr Ala Lys Arg Lys Ile
               100                 105                 110
   gaa cag att caa ttt tac ggc ggc cac tgt cat ttt gtg gag agc gcc       384
   Glu Gln Ile Gln Phe Tyr Gly Gly His Cys His Phe Val Glu Ser Ala
           115                 120                 125
   tgc gaa att tat gcc gcc tcc gag cgg ctg gcg cat gaa ctg aac ggt       432
   Cys Glu Ile Tyr Ala Ala Ser Glu Arg Leu Ala His Glu Leu Asn Gly
       130                 135                 140
   cac tat atg gac cag ttt acc tac gca gaa cga gct acc gac tgg cgg       480
   His Tyr Met Asp Gln Phe Thr Tyr Ala Glu Arg Ala Thr Asp Trp Arg
   145                 150                 155                 160
   ggc aac aat aat att gcc gac agt att ttc cgc cag atg cgc aac gaa       528
   Gly Asn Asn Asn Ile Ala Asp Ser Ile Phe Arg Gln Met Arg Asn Glu
                   165                 170                 175
   ctc cat ccg gta ccg cga ttt atc gtc atg agc gcc gga acg ggc gga       576
   Leu His Pro Val Pro Arg Phe Ile Val Met Ser Ala Gly Thr Gly Gly
               180                 185                 190
   acc tct gcg acg att ggg cgc tat att cgc tgc cag ggc tat gat act       624
   Thr Ser Ala Thr Ile Gly Arg Tyr Ile Arg Cys Gln Gly Tyr Asp Thr
           195                 200                 205
   cag ctc atg gtg gtg gat ccg gaa aat tcc gtc ttt tta cct tac tgg       672
   Gln Leu Met Val Val Asp Pro Glu Asn Ser Val Phe Leu Pro Tyr Trp
       210                 215                 220
   cag gat cgc gac gct tca tta cgc agc ccg gta ggc agt aaa att gaa       720
   Gln Asp Arg Asp Ala Ser Leu Arg Ser Pro Val Gly Ser Lys Ile Glu
   225                 230                 235                 240
   gga att ggt cgt ccg cga gta gag cct tct ttt att ccg gat gtc gtg       768
   Gly Ile Gly Arg Pro Arg Val Glu Pro Ser Phe Ile Pro Asp Val Val
                   245                 250                 255
   gat gag atg ctg cgc gtg ccg gac gcc gcc agc gtt gcc acc gca cac       816
   Asp Glu Met Leu Arg Val Pro Asp Ala Ala Ser Val Ala Thr Ala His
               260                 265                 270
   tgg ctg gaa acg cag cta ggc cgt aaa gtc ggc gcc tct acc ggc acg       864
   Trp Leu Glu Thr Gln Leu Gly Arg Lys Val Gly Ala Ser Thr Gly Thr
           275                 280                 285
   aat atg tgg gga gct ctg cag ctt gcg gcc cgg atg cgc gag gct gga       912
   Asn Met Trp Gly Ala Leu Gln Leu Ala Ala Arg Met Arg Glu Ala Gly
       290                 295                 300
   gaa acc ggc gct atc gtg acg tta ttg tgc gat agc ggc gat cgc tat       960
   Glu Thr Gly Ala Ile Val Thr Leu Leu Cys Asp Ser Gly Asp Arg Tyr
   305                 310                 315                 320
   ctg gat acc tat tac cat ccg gcc tgg gtt agc gac cat atc ggc gat      1008
   Leu Asp Thr Tyr Tyr His Pro Ala Trp Val Ser Asp His Ile Gly Asp
                   325                 330                 335
   ttg acg ccg tgg tca gcg gcc atc gca aaa tta ctt acc ggc gac tag      1056
   Leu Thr Pro Trp Ser Ala Ala Ile Ala Lys Leu Leu Thr Gly Asp
               340                 345                 350
<210> 40
   <211> 351
   <212> PRT
   <213> Salmonella typhimurium
<400> 40
   Met Met Ser Ser Asn Trp Val Lys Asn Ala Ile Asn Glu Ile Asn Ala
   1               5                   10                  15
   Asp His Gln Arg Ser Ala Asp Thr His Leu Ile Arg Leu Pro Leu Ser
               20                  25                  30
   Ala Phe Pro Gly Ile Gln Leu Tyr Leu Lys Asp Glu Ser Thr His Pro
           35                  40                  45
   Thr Gly Ser Leu Lys His Arg Leu Ala Arg Ser Leu Phe Leu Tyr Gly
       50                  55                  60
   Leu Cys Asn Gly Trp Ile Lys Glu Gly Thr Pro Ile Ile Glu Ala Ser
   65                  70                  75                  80
   Ser Gly Ser Thr Ala Ile Ser Glu Ala Trp Phe Ala Arg Leu Leu Gly
                   85                  90                  95
   Leu Pro Phe Ile Ala Val Met Pro Ala Cys Thr Ala Lys Arg Lys Ile
               100                 105                 110
   Glu Gln Ile Gln Phe Tyr Gly Gly His Cys His Phe Val Glu Ser Ala
           115                 120                 125
   Cys Glu Ile Tyr Ala Ala Ser Glu Arg Leu Ala His Glu Leu Asn Gly
       130                 135                 140
   His Tyr Met Asp Gln Phe Thr Tyr Ala Glu Arg Ala Thr Asp Trp Arg
   145                 150                 155                 160
   Gly Asn Asn Asn Ile Ala Asp Ser Ile Phe Arg Gln Met Arg Asn Glu
                   165                 170                 175
   Leu His Pro Val Pro Arg Phe Ile Val Met Ser Ala Gly Thr Gly Gly
               180                 185                 190
   Thr Ser Ala Thr Ile Gly Arg Tyr Ile Arg Cys Gln Gly Tyr Asp Thr
           195                 200                 205
   Gln Leu Met Val Val Asp Pro Glu Asn Ser Val Phe Leu Pro Tyr Trp
       210                 215                 220
   Gln Asp Arg Asp Ala Ser Leu Arg Ser Pro Val Gly Ser Lys Ile Glu
   225                 230                 235                 240
   Gly Ile Gly Arg Pro Arg Val Glu Pro Ser Phe Ile Pro Asp Val Val
                   245                 250                 255
   Asp Glu Met Leu Arg Val Pro Asp Ala Ala Ser Val Ala Thr Ala His
               260                 265                 270
   Trp Leu Glu Thr Gln Leu Gly Arg Lys Val Gly Ala Ser Thr Gly Thr
           275                 280                 285
   Asn Met Trp Gly Ala Leu Gln Leu Ala Ala Arg Met Arg Glu Ala Gly
       290                 295                 300
   Glu Thr Gly Ala Ile Val Thr Leu Leu Cys Asp Ser Gly Asp Arg Tyr
   305                 310                 315                 320
   Leu Asp Thr Tyr Tyr His Pro Ala Trp Val Ser Asp His Ile Gly Asp
                   325                 330                 335
   Leu Thr Pro Trp Ser Ala Ala Ile Ala Lys Leu Leu Thr Gly Asp
               340                 345                 350
<210> 41
   <211> 1044
   <212> DNA
   <213> Serratia proteamaculans
<220>
   <221> CDS
   <222> (1)..(1044)
<400> 41
   atg atg aca aat tct tgg gta aaa tat gct atc ggt gaa ata gaa gcg 48
   Met Met Thr Asn Ser Trp Val Lys Tyr Ala Ile Gly Glu Ile Glu Ala
   1 5 10 15
   gat ttt caa cgc tcc gcc gat act cac ctg atc cgc ctg aac ctg ccg 96
   Asp Phe Gln Arg Ser Ala Asp Thr His Leu Ile Arg Leu Asn Leu Pro
   20 25 30
   gaa ttt ccc ggc atc tgc ctg tac ctg aaa gat gaa agc act cac ccg 144
   Glu Phe Pro Gly Ile Cys Leu Tyr Leu Lys Asp Glu Ser Thr His Pro
   35 40 45
   acc ggc agc ctg aag cac cgg ctg gcg cgt tca cta ttt ttg tat ggt 192
   Thr Gly Ser Leu Lys His Arg Leu Ala Arg Ser Leu Phe Leu Tyr Gly
   50 55 60
   ctg tgt aac ggc tgg atc acc gaa aac acc acg att atc gag gcc tcc 240
   Leu Cys Asn Gly Trp Ile Thr Glu Asn Thr Thr Ile Ile Glu Ala Ser
   65 70 75 80
   tcc ggc agt acc gcg gta tcc gaa gct tac ttc gcc cgg ctg att ggc 288
   Ser Gly Ser Thr Ala Val Ser Glu Ala Tyr Phe Ala Arg Leu Ile Gly
   85 90 95
   ctg ccg ttt att gcc gtc atg cct tcc tgt acc gca cgc cgc aag gtt 336
   Leu Pro Phe Ile Ala Val Met Pro Ser Cys Thr Ala Arg Arg Lys Val
   100 105 110
   gag cag atc gcg ttc tac ggc ggt cgc tgt cac ttc gtc gac cat gcg 384
   Glu Gln Ile Ala Phe Tyr Gly Gly Arg Cys His Phe Val Asp His Ala
   115 120 125
   gcg caa att tac gcc gtc tcc gaa cag ttg gcg aaa gag ctg aac ggc 432
   Ala Gln Ile Tyr Ala Val Ser Glu Gln Leu Ala Lys Glu Leu Asn Gly
   130 135 140
   cac tat atg gat cag ttc acc tac gct gaa cgg gcc acc gac tgg cgc 480
   His Tyr Met Asp Gln Phe Thr Tyr Ala Glu Arg Ala Thr Asp Trp Arg
   145 150 155 160
   ggc aac aac aac atc gcc gac agc atc ttc cgc cag atg gaa cgc gaa 528
   Gly Asn Asn Asn Ile Ala Asp Ser Ile Phe Arg Gln Met Glu Arg Glu
   165 170 175
   cct ttc ccg gta ccc aag cat atc gta atg agt gcc ggt acc ggt ggc 576
   Pro Phe Pro Val Pro Lys His Ile Val Met Ser Ala Gly Thr Gly Gly
   180 185 190
   acc tct gcg acc ctg ggc cgc tat atc cgc tat cag ggg cac gac acc 624
   Thr Ser Ala Thr Leu Gly Arg Tyr Ile Arg Tyr Gln Gly His Asp Thr
   195 200 205
   cgc ctg acg gtg gtc gat ccg gaa aat tcg gtg ttc tat gat tgc ttc 672
   Arg Leu Thr Val Val Asp Pro Glu Asn Ser Val Phe Tyr Asp Cys Phe
   210 215 220
   cac cag cat gac cgc acc ctt atc ggc agt tgc ggc agc cgt atc gag 720
   His Gln His Asp Arg Thr Leu Ile Gly Ser Cys Gly Ser Arg Ile Glu
   225 230 235 240
   ggc att ggc cgc cca cgc gcc gag cca tcc ttt att ccg tcg gtg gtc 768
   Gly Ile Gly Arg Pro Arg Ala Glu Pro Ser Phe Ile Pro Ser Val Val
   245 250 255
   gac agc atg atc cgc gtg ccg gat gcc gcc agc gtc gcc act atc cac 816
   Asp Ser Met Ile Arg Val Pro Asp Ala Ala Ser Val Ala Thr Ile His
   260 265 270
   tgg ctg gaa acg gtg ctc gga cgc aag gtc ggg gcc tcc acc ggc acc 864
   Trp Leu Glu Thr Val Leu Gly Arg Lys Val Gly Ala Ser Thr Gly Thr
   275 280 285
   aac gtg tgg ggt gcg ctg ctg ttg gcg aag caa atg cgg gaa aac ggc 912
   Asn Val Trp Gly Ala Leu Leu Leu Ala Lys Gln Met Arg Glu Asn Gly
   290 295 300
   gag cag ggt tcg att gtg act ctg ctg tgc gac agc ggt gag cgc tac 960
   Glu Gln Gly Ser Ile Val Thr Leu Leu Cys Asp Ser Gly Glu Arg Tyr
   305 310 315 320
   ctg gac acc tat tac aac ccg gaa tgg gtc aga aac aat atc ggt gat 1008
   Leu Asp Thr Tyr Tyr Asn Pro Glu Trp Val Arg Asn Asn Ile Gly Asp
   325 330 335
   ctg acg cct tat ctg gcg cag tta gca gat ttg taa 1044
   Leu Thr Pro Tyr Leu Ala Gln Leu Ala Asp Leu
   340 345
<210> 42
   <211> 347
   <212> PRT
   <213> Serratia proteamaculans
<400> 42
   Met Met Thr Asn Ser Trp Val Lys Tyr Ala Ile Gly Glu Ile Glu Ala
   1               5                   10                  15
   Asp Phe Gln Arg Ser Ala Asp Thr His Leu Ile Arg Leu Asn Leu Pro
               20                  25                  30
   Glu Phe Pro Gly Ile Cys Leu Tyr Leu Lys Asp Glu Ser Thr His Pro
           35                  40                  45
   Thr Gly Ser Leu Lys His Arg Leu Ala Arg Ser Leu Phe Leu Tyr Gly
       50                  55                  60
   Leu Cys Asn Gly Trp Ile Thr Glu Asn Thr Thr Ile Ile Glu Ala Ser
   65                  70                  75                  80
   Ser Gly Ser Thr Ala Val Ser Glu Ala Tyr Phe Ala Arg Leu Ile Gly
                   85                  90                  95
   Leu Pro Phe Ile Ala Val Met Pro Ser Cys Thr Ala Arg Arg Lys Val
               100                 105                 110
   Glu Gln Ile Ala Phe Tyr Gly Gly Arg Cys His Phe Val Asp His Ala
           115                 120                 125
   Ala Gln Ile Tyr Ala Val Ser Glu Gln Leu Ala Lys Glu Leu Asn Gly
       130                 135                 140
   His Tyr Met Asp Gln Phe Thr Tyr Ala Glu Arg Ala Thr Asp Trp Arg
   145                 150                 155                 160
   Gly Asn Asn Asn Ile Ala Asp Ser Ile Phe Arg Gln Met Glu Arg Glu
                   165                 170                 175
   Pro Phe Pro Val Pro Lys His Ile Val Met Ser Ala Gly Thr Gly Gly
               180                 185                 190
   Thr Ser Ala Thr Leu Gly Arg Tyr Ile Arg Tyr Gln Gly His Asp Thr
           195                 200                 205
   Arg Leu Thr Val Val Asp Pro Glu Asn Ser Val Phe Tyr Asp Cys Phe
       210                 215                 220
   His Gln His Asp Arg Thr Leu Ile Gly Ser Cys Gly Ser Arg Ile Glu
   225                 230                 235                 240
   Gly Ile Gly Arg Pro Arg Ala Glu Pro Ser Phe Ile Pro Ser Val Val
                   245                 250                 255
   Asp Ser Met Ile Arg Val Pro Asp Ala Ala Ser Val Ala Thr Ile His
               260                 265                 270
   Trp Leu Glu Thr Val Leu Gly Arg Lys Val Gly Ala Ser Thr Gly Thr
           275                 280                 285
   Asn Val Trp Gly Ala Leu Leu Leu Ala Lys Gln Met Arg Glu Asn Gly
       290                 295                 300
   Glu Gln Gly Ser Ile Val Thr Leu Leu Cys Asp Ser Gly Glu Arg Tyr
   305                 310                 315                 320
   Leu Asp Thr Tyr Tyr Asn Pro Glu Trp Val Arg Asn Asn Ile Gly Asp
                   325                 330                 335
   Leu Thr Pro Tyr Leu Ala Gln Leu Ala Asp Leu
               340                 345
<210> 43
   <211> 1077
   <212> DNA
   <213> Erwinia carotovora
<220>
   <221> CDS
   <222> (1)..(1077)
<400> 43
   atg acc aat gcc tgg gtt aaa aat gcc atc agc gct atc gaa gca gat        48
   Met Thr Asn Ala Trp Val Lys Asn Ala Ile Ser Ala Ile Glu Ala Asp
   1               5                   10                  15
   ttt cag cgt tcg gct gat acg cat ctt ata cgt ctg acc ctg ccg gat        96
   Phe Gln Arg Ser Ala Asp Thr His Leu Ile Arg Leu Thr Leu Pro Asp
               20                  25                  30
   tat ccc ggt atc tat ttt tat ctc aaa gat gaa agc aca cac ccc agc       144
   Tyr Pro Gly Ile Tyr Phe Tyr Leu Lys Asp Glu Ser Thr His Pro Ser
           35                  40                  45
   ggt agc ctg aag cat cgc ctg gcg cgc tcc ttg ttt ctc tac ggg ctg       192
   Gly Ser Leu Lys His Arg Leu Ala Arg Ser Leu Phe Leu Tyr Gly Leu
       50                  55                  60
   tgc aac ggt tgg att aaa gaa ggt acg cca att atc gaa gcg tca tct       240
   Cys Asn Gly Trp Ile Lys Glu Gly Thr Pro Ile Ile Glu Ala Ser Ser
   65                  70                  75                  80
   ggc agt acc gcc gta tca gaa gcc tat ttt gca cgc ttg ctt ggc tta       288
   Gly Ser Thr Ala Val Ser Glu Ala Tyr Phe Ala Arg Leu Leu Gly Leu
                   85                  90                  95
   ccg ttt att gcc gtc atg ccg tcc tgc acc gca aaa aga aaa ata gag       336
   Pro Phe Ile Ala Val Met Pro Ser Cys Thr Ala Lys Arg Lys Ile Glu
               100                 105                 110
   caa atc gct ttc tat ggc ggg cgc tgt cat ttt gtt gag cag gcg gga       384
   Gln Ile Ala Phe Tyr Gly Gly Arg Cys His Phe Val Glu Gln Ala Gly
           115                 120                 125
   caa atc tat gct gca tcc gaa cag ctg gcg caa gag atg aac ggc cac       432
   Gln Ile Tyr Ala Ala Ser Glu Gln Leu Ala Gln Glu Met Asn Gly His
       130                 135                 140
   tat atg gac cag ttc acc tac gcg gaa cgc gct acc gac tgg cgt ggc       480
   Tyr Met Asp Gln Phe Thr Tyr Ala Glu Arg Ala Thr Asp Trp Arg Gly
   145                 150                 155                 160
   aat aac aat att gcc gac agc att tac cgt caa atg gca cgt gaa ccc       528
   Asn Asn Asn Ile Ala Asp Ser Ile Tyr Arg Gln Met Ala Arg Glu Pro
                   165                 170                 175
   cat ccc gta ccg gac tac att gtg atg agt gcc ggt acg ggc ggc acg       576
   His Pro Val Pro Asp Tyr Ile Val Met Ser Ala Gly Thr Gly Gly Thr
               180                 185                 190
   tca gca acg ctc ggt cgc tac att cgc tat cag ggg ttg gat acg caa       624
   Ser Ala Thr Leu Gly Arg Tyr Ile Arg Tyr Gln Gly Leu Asp Thr Gln
           195                 200                 205
   ctg gtc gtc gtc gat cca gaa aac tcg gtc ttc tac gac tgc tat cag       672
   Leu Val Val Val Asp Pro Glu Asn Ser Val Phe Tyr Asp Cys Tyr Gln
       210                 215                 220
   aaa cag gat cgt tcc atc att ggc cca tgc ggc agt cgg ata gaa ggt       720
   Lys Gln Asp Arg Ser Ile Ile Gly Pro Cys Gly Ser Arg Ile Glu Gly
   225                 230                 235                 240
   atc ggc cgc ccg cgt gcc gag cct tct ttt att cca ggc gtc att gat       768
   Ile Gly Arg Pro Arg Ala Glu Pro Ser Phe Ile Pro Gly Val Ile Asp
                   245                 250                 255
   agc atg atc aaa gtg ccg gat gcc gcc agc atc gca acg ctg tac tgg       816
   Ser Met Ile Lys Val Pro Asp Ala Ala Ser Ile Ala Thr Leu Tyr Trp
               260                 265                 270
   ctg gaa agc gta tta ggc cgc aaa gca ggc gcc tct act ggc acg aat       864
   Leu Glu Ser Val Leu Gly Arg Lys Ala Gly Ala Ser Thr Gly Thr Asn
           275                 280                 285
   gtt tgg ggt atg ctg caa ttg gcc aaa gaa atg gtt aac cat ggc caa       912
   Val Trp Gly Met Leu Gln Leu Ala Lys Glu Met Val Asn His Gly Gln
       290                 295                 300
   aaa ggc gcg att gtg acc ctg tta tgt gac agc ggc gaa cgc tat ctg       960
   Lys Gly Ala Ile Val Thr Leu Leu Cys Asp Ser Gly Glu Arg Tyr Leu
   305                 310                 315                 320
   gac acc tac tac aac agt agc tgg gta gaa aaa aac atc ggc gac att      1008
   Asp Thr Tyr Tyr Asn Ser Ser Trp Val Glu Lys Asn Ile Gly Asp Ile
                   325                 330                 335
   agc cct tac ctc aac gcc ctg aac aac ccg gag atg tcc ttt ctc ata      1056
   Ser Pro Tyr Leu Asn Ala Leu Asn Asn Pro Glu Met Ser Phe Leu Ile
               340                 345                 350
   ccg gaa ccc agt ctg gcg tga                                          1077
   Pro Glu Pro Ser Leu Ala
           355
<210> 44
   <211> 358
   <212> PRT
   <213> Erwinia carotovora
<400> 44
   Met Thr Asn Ala Trp Val Lys Asn Ala Ile Ser Ala Ile Glu Ala Asp
   1               5                   10                  15
   Phe Gln Arg Ser Ala Asp Thr His Leu Ile Arg Leu Thr Leu Pro Asp
               20                  25                  30
   Tyr Pro Gly Ile Tyr Phe Tyr Leu Lys Asp Glu Ser Thr His Pro Ser
           35                  40                  45
   Gly Ser Leu Lys His Arg Leu Ala Arg Ser Leu Phe Leu Tyr Gly Leu
       50                  55                  60
   Cys Asn Gly Trp Ile Lys Glu Gly Thr Pro Ile Ile Glu Ala Ser Ser
   65                  70                  75                  80
   Gly Ser Thr Ala Val Ser Glu Ala Tyr Phe Ala Arg Leu Leu Gly Leu
                   85                  90                  95
   Pro Phe Ile Ala Val Met Pro Ser Cys Thr Ala Lys Arg Lys Ile Glu
               100                 105                 110
   Gln Ile Ala Phe Tyr Gly Gly Arg Cys His Phe Val Glu Gln Ala Gly
           115                 120                 125
   Gln Ile Tyr Ala Ala Ser Glu Gln Leu Ala Gln Glu Met Asn Gly His
       130                 135                 140
   Tyr Met Asp Gln Phe Thr Tyr Ala Glu Arg Ala Thr Asp Trp Arg Gly
   145                 150                 155                 160
   Asn Asn Asn Ile Ala Asp Ser Ile Tyr Arg Gln Met Ala Arg Glu Pro
                   165                 170                 175
   His Pro Val Pro Asp Tyr Ile Val Met Ser Ala Gly Thr Gly Gly Thr
               180                 185                 190
   Ser Ala Thr Leu Gly Arg Tyr Ile Arg Tyr Gln Gly Leu Asp Thr Gln
           195                 200                 205
   Leu Val Val Val Asp Pro Glu Asn Ser Val Phe Tyr Asp Cys Tyr Gln
       210                 215                 220
   Lys Gln Asp Arg Ser Ile Ile Gly Pro Cys Gly Ser Arg Ile Glu Gly
   225                 230                 235                 240
   Ile Gly Arg Pro Arg Ala Glu Pro Ser Phe Ile Pro Gly Val Ile Asp
                   245                 250                 255
   Ser Met Ile Lys Val Pro Asp Ala Ala Ser Ile Ala Thr Leu Tyr Trp
               260                 265                 270
   Leu Glu Ser Val Leu Gly Arg Lys Ala Gly Ala Ser Thr Gly Thr Asn
           275                 280                 285
   Val Trp Gly Met Leu Gln Leu Ala Lys Glu Met Val Asn His Gly Gln
       290                 295                 300
   Lys Gly Ala Ile Val Thr Leu Leu Cys Asp Ser Gly Glu Arg Tyr Leu
   305                 310                 315                 320
   Asp Thr Tyr Tyr Asn Ser Ser Trp Val Glu Lys Asn Ile Gly Asp Ile
                   325                 330                 335
   Ser Pro Tyr Leu Asn Ala Leu Asn Asn Pro Glu Met Ser Phe Leu Ile
               340                 345                 350
   Pro Glu Pro Ser Leu Ala
           355
<210> 45
   <211> 1068
   <212> DNA
   <213> vibrio cholerae
<220>
   <221> CDS
   <222> (1)..(1068)
<400> 45
   atg tgt acc gac cat cag tgg att aac agt gca att cgt aaa att gaa 48
   Met Cys Thr Asp His Gln Trp Ile Asn Ser Ala Ile Arg Lys Ile Glu
   1 5 10 15
   gca gac tat caa cgc tcc gcc gac acc cat ctg att aaa ctc gat tta 96
   Ala Asp Tyr Gln Arg Ser Ala Asp Thr His Leu Ile Lys Leu Asp Leu
   20 25 30
   cct tgc gtc tct ggc gtc gat atc tat ctg aaa gat gaa agt acc cac 144
   Pro Cys Val Ser Gly Val Asp Ile Tyr Leu Lys Asp Glu Ser Thr His
   35 40 45
   ccc acc ggt tct ctc aaa cac cgt ctc gcc cgt tca ctg ttt tta tat 192
   Pro Thr Gly Ser Leu Lys His Arg Leu Ala Arg Ser Leu Phe Leu Tyr
   50 55 60
   ggg ctg tgt aat ggc tgg att ggc cca gaa act acc att att gaa tcc 240
   Gly Leu Cys Asn Gly Trp Ile Gly Pro Glu Thr Thr Ile Ile Glu Ser
   65 70 75 80
   tca tct ggc agt act gcc gta tcg gaa gct tac ttt gct cgc ttg ctt 288
   Ser Ser Gly Ser Thr Ala Val Ser Glu Ala Tyr Phe Ala Arg Leu Leu
   85 90 95
   ggc cta ccc ttt att gcc gtg atg cca aag tgc acc gcg cgt aaa aaa 336
   Gly Leu Pro Phe Ile Ala Val Met Pro Lys Cys Thr Ala Arg Lys Lys
   100 105 110
   atc gaa cag atc cag ttc tat ggg ggc aag gcg cat ctt gta gat cgc 384
   Ile Glu Gln Ile Gln Phe Tyr Gly Gly Lys Ala His Leu Val Asp Arg
   115 120 125
   tcc gat caa att tac gca gaa tct cat cgt ctc gcg aaa gag ctg aaa 432
   Ser Asp Gln Ile Tyr Ala Glu Ser His Arg Leu Ala Lys Glu Leu Lys
   130 135 140
   ggc cac tac atg gat cag ttt act tac gct gaa cga gcc acc gac tgg 480
   Gly His Tyr Met Asp Gln Phe Thr Tyr Ala Glu Arg Ala Thr Asp Trp
   145 150 155 160
   cgt ggt aac agc aac atc gct gac agt att ttt agc cag atg cag atg 528
   Arg Gly Asn Ser Asn Ile Ala Asp Ser Ile Phe Ser Gln Met Gln Met
   165 170 175
   gaa caa cac ccc gtt cca agc tgg att gtg atg agc ccg ggg acg gga 576
   Glu Gln His Pro Val Pro Ser Trp Ile Val Met Ser Pro Gly Thr Gly
   180 185 190
   ggc acc tca gcc act atc ggc cgc ttt att cgc tac caa caa tac aac 624
   Gly Thr Ser Ala Thr Ile Gly Arg Phe Ile Arg Tyr Gln Gln Tyr Asn
   195 200 205
   acc aaa ctg tgt gtg gtt gac ccg gaa aac tcg gtt ttt tat gac tac 672
   Thr Lys Leu Cys Val Val Asp Pro Glu Asn Ser Val Phe Tyr Asp Tyr
   210 215 220
   tac cgc acg cga aat gcc gag cta acc cgt gac tgc ggc agc aaa att 720
   Tyr Arg Thr Arg Asn Ala Glu Leu Thr Arg Asp Cys Gly Ser Lys Ile
   225 230 235 240
   gaa ggc att ggc cgc cca aga gtc gag ccg agc ttt att cct gat gtg 768
   Glu Gly Ile Gly Arg Pro Arg Val Glu Pro Ser Phe Ile Pro Asp Val
   245 250 255
   gta gat gaa atg cgt gcg atc cct gat gcc gcg agt gta gcg acc atc 816
   Val Asp Glu Met Arg Ala Ile Pro Asp Ala Ala Ser Val Ala Thr Ile
   260 265 270
   tac tgg ttg gag aaa att ctt ggc cgt aaa gca ggc gcc tcc act ggc 864
   Tyr Trp Leu Glu Lys Ile Leu Gly Arg Lys Ala Gly Ala Ser Thr Gly
   275 280 285
   act aac ctg tat ggc gcg ctg caa ctc gcc tgc gaa atg aag cgc cgc 912
   Thr Asn Leu Tyr Gly Ala Leu Gln Leu Ala Cys Glu Met Lys Arg Arg
   290 295 300
   ggt gag caa ggt tcg att gtc acg ctc ttg tgc gat agc ggt gaa cgc 960
   Gly Glu Gln Gly Ser Ile Val Thr Leu Leu Cys Asp Ser Gly Glu Arg
   305 310 315 320
   tac ctt gat acc tat tac aac tct gag tgg gtc aaa aac aac att ggc 1008
   Tyr Leu Asp Thr Tyr Tyr Asn Ser Glu Trp Val Lys Asn Asn Ile Gly
   325 330 335
   gat tta agt aaa tat ctg cac aag ttg gaa aca ttc tcc gcg aca ggc 1056
   Asp Leu Ser Lys Tyr Leu His Lys Leu Glu Thr Phe Ser Ala Thr Gly
   340 345 350
   tgt ttg gat taa 1068
   Cys Leu Asp
   355
<210> 46
   <211> 355
   <212> PRT
   <213> vibrio cholerae
<400> 46
   Met Cys Thr Asp His Gln Trp Ile Asn Ser Ala Ile Arg Lys Ile Glu
   1               5                   10                  15
   Ala Asp Tyr Gln Arg Ser Ala Asp Thr His Leu Ile Lys Leu Asp Leu
               20                  25                  30
   Pro Cys Val Ser Gly Val Asp Ile Tyr Leu Lys Asp Glu Ser Thr His
           35                  40                  45
   Pro Thr Gly Ser Leu Lys His Arg Leu Ala Arg Ser Leu Phe Leu Tyr
       50                  55                  60
   Gly Leu Cys Asn Gly Trp Ile Gly Pro Glu Thr Thr Ile Ile Glu Ser
   65                  70                  75                  80
   Ser Ser Gly Ser Thr Ala Val Ser Glu Ala Tyr Phe Ala Arg Leu Leu
                   85                  90                  95
   Gly Leu Pro Phe Ile Ala Val Met Pro Lys Cys Thr Ala Arg Lys Lys
               100                 105                 110
   Ile Glu Gln Ile Gln Phe Tyr Gly Gly Lys Ala His Leu Val Asp Arg
           115                 120                 125
   Ser Asp Gln Ile Tyr Ala Glu Ser His Arg Leu Ala Lys Glu Leu Lys
       130                 135                 140
   Gly His Tyr Met Asp Gln Phe Thr Tyr Ala Glu Arg Ala Thr Asp Trp
   145                 150                 155                 160
   Arg Gly Asn Ser Asn Ile Ala Asp Ser Ile Phe Ser Gln Met Gln Met
                   165                 170                 175
   Glu Gln His Pro Val Pro Ser Trp Ile Val Met Ser Pro Gly Thr Gly
               180                 185                 190
   Gly Thr Ser Ala Thr Ile Gly Arg Phe Ile Arg Tyr Gln Gln Tyr Asn
           195                 200                 205
   Thr Lys Leu Cys Val Val Asp Pro Glu Asn Ser Val Phe Tyr Asp Tyr
       210                 215                 220
   Tyr Arg Thr Arg Asn Ala Glu Leu Thr Arg Asp Cys Gly Ser Lys Ile
   225                 230                 235                 240
   Glu Gly Ile Gly Arg Pro Arg Val Glu Pro Ser Phe Ile Pro Asp Val
                   245                 250                 255
   Val Asp Glu Met Arg Ala Ile Pro Asp Ala Ala Ser Val Ala Thr Ile
               260                 265                 270
   Tyr Trp Leu Glu Lys Ile Leu Gly Arg Lys Ala Gly Ala Ser Thr Gly
           275                 280                 285
   Thr Asn Leu Tyr Gly Ala Leu Gln Leu Ala Cys Glu Met Lys Arg Arg
       290                 295                 300
   Gly Glu Gln Gly Ser Ile Val Thr Leu Leu Cys Asp Ser Gly Glu Arg
   305                 310                 315                 320
   Tyr Leu Asp Thr Tyr Tyr Asn Ser Glu Trp Val Lys Asn Asn Ile Gly
                   325                 330                 335
   Asp Leu Ser Lys Tyr Leu His Lys Leu Glu Thr Phe Ser Ala Thr Gly
               340                 345                 350
   Cys Leu Asp
           355
<210> 47
   <211> 1095
   <212> DNA
   <213> Pseudomonas fluorescens
<220>
   <221> CDS
   <222> (1)..(1095)
<400> 47 <210> 48
   <211> 364
   <212> PRT
   <213> Pseudomonas fluorescens
<400> 48
<210> 49
   <211> 1125
   <212> DNA <213> Streptomyces coelicolor
<220>

   <221> CDS
   <222> (1)..(1125)
<400> 49
   gtg agc act gac cga cag acc ggc acc ggc acc acc ctc gac gtc gac 48
   Val Ser Thr Asp Arg Gln Thr Gly Thr Gly Thr Thr Leu Asp Val Asp
   1 5 10 15
   cgc agc gac gcc tcc tac cgt gcc tgg ctg aaa gag gcc gtc cgc aag 96
   Arg Ser Asp Ala Ser Tyr Arg Ala Trp Leu Lys Glu Ala Val Arg Lys
   20 25 30
   gtc cag gcc gac gcg aac cgc tcg gcc gac acc cac ctg ctc ctg ttc 144
   Val Gln Ala Asp Ala Asn Arg Ser Ala Asp Thr His Leu Leu Leu Phe
   35 40 45
   ccg ctc ccc gag cac tgg ggc atc gac ctg tac ctg aag gac gag tcg 192
   Pro Leu Pro Glu His Trp Gly Ile Asp Leu Tyr Leu Lys Asp Glu Ser
   50 55 60
   acc cac ccg acc ggc agc ctc aag cac cgg ctc gcc cgc tcc ctg ttc 240
   Thr His Pro Thr Gly Ser Leu Lys His Arg Leu Ala Arg Ser Leu Phe
   65 70 75 80
   ctc tac ggc ctg tgc aac ggc tgg atc cgg ccg gac cgc ccg gtg atc 288
   Leu Tyr Gly Leu Cys Asn Gly Trp Ile Arg Pro Asp Arg Pro Val Ile
   85 90 95
   gag gcg tcc agc ggc tcg acg gcc gtc tcc gag gcc tat ttc gcc aag 336
   Glu Ala Ser Ser Gly Ser Thr Ala Val Ser Glu Ala Tyr Phe Ala Lys
   100 105 110
   ctg gtc ggc gtc ccc ttc atc gcc gtc atg ccc cgg acg acg agc gcc 384
   Leu Val Gly Val Pro Phe Ile Ala Val Met Pro Arg Thr Thr Ser Ala
   115 120 125
   gag aag atc cgg ctg atc gag ttc cac ggg gga cgg tgc cac ttc gtg 432
   Glu Lys Ile Arg Leu Ile Glu Phe His Gly Gly Arg Cys His Phe Val
   130 135 140
   gac gac tcc cgg cgg atg tac gag gag tcg gcc cgc ctc gcg gcg gag 480
   Asp Asp Ser Arg Arg Met Tyr Glu Glu Ser Ala Arg Leu Ala Ala Glu
   145 150 155 160
   acc ggc ggc cac tac atg gac cag ttc acc tac gcc gaa cgc gcc acg 528
   Thr Gly Gly His Tyr Met Asp Gln Phe Thr Tyr Ala Glu Arg Ala Thr
   165 170 175
   gac tgg cgc ggc aac aac aac atc gcc gag tcg atc ttc cgt cag ctg 576
   Asp Trp Arg Gly Asn Asn Asn Ile Ala Glu Ser Ile Phe Arg Gln Leu
   180 185 190
   cgg ctg gaa cgg tat ccc gag ccg tcc tgg atc gtc gcc acg gcc ggc 624
   Arg Leu Glu Arg Tyr Pro Glu Pro Ser Trp Ile Val Ala Thr Ala Gly
   195 200 205
   acc ggc ggc acc tcg gcg acc ctc gcc cgc tac gtc cac tac atg cag 672
   Thr Gly Gly Thr Ser Ala Thr Leu Ala Arg Tyr Val His Tyr Met Gln
   210 215 220
   tac gac acc cgc gtc tgc gtc gcc gac ccg gac aac tcc tgc ttc ttc 720
   Tyr Asp Thr Arg Val Cys Val Ala Asp Pro Asp Asn Ser Cys Phe Phe
   225 230 235 240
   gag ggt tgg acc acc ggc gac ccc gac gtg gcc tgc gac cgc ggc tcc 768
   Glu Gly Trp Thr Thr Gly Asp Pro Asp Val Ala Cys Asp Arg Gly Ser
   245 250 255
   cgc atc gag ggc atc ggc cgg ccc cgg atg gaa ccg agc ttc gta ccc 816
   Arg Ile Glu Gly Ile Gly Arg Pro Arg Met Glu Pro Ser Phe Val Pro
   260 265 270
   ggc gcg atc gac cgc atg atg aag gtg ccg gac gcg gcc agc gtc gcg 864
   Gly Ala Ile Asp Arg Met Met Lys Val Pro Asp Ala Ala Ser Val Ala
   275 280 285
   gcc gtg cgg tcc ctg gag cgg gcc atc ggc cgc agg gcg ggc ggc tcc 912
   Ala Val Arg Ser Leu Glu Arg Ala Ile Gly Arg Arg Ala Gly Gly Ser
   290 295 300
   acc ggc acc ggg ctg tgg agc gcg ttg aag atc gtc gcc gag atg gtg 960
   Thr Gly Thr Gly Leu Trp Ser Ala Leu Lys Ile Val Ala Glu Met Val
   305 310 315 320
   gcc gcg ggg gag cgg ggc agc gtc gtg aca ctg ctg tgc gat ccg ggg 1008
   Ala Ala Gly Glu Arg Gly Ser Val Val Thr Leu Leu Cys Asp Pro Gly
                   325                 330                 335
   gac cgg tac ctc gac aag tac tac tcg gac gcc tgg ctc gcc gaa cag      1056
   Asp Arg Tyr Leu Asp Lys Tyr Tyr Ser Asp Ala Trp Leu Ala Glu Gln
               340                 345                 350
   ggc ctc gac atc gag ccg tac acg gcg gcg atc gag tcc ctg ctg cgc      1104
   Gly Leu Asp Ile Glu Pro Tyr Thr Ala Ala Ile Glu Ser Leu Leu Arg
           355                 360                 365
   acc ggc agc ctg ctc gac tga                                          1125
   Thr Gly Ser Leu Leu Asp
       370
<210> 50
   <211> 374
   <212> PRT
   <213> Streptomyces coelicolor
<400> 50
   Val Ser Thr Asp Arg Gln Thr Gly Thr Gly Thr Thr Leu Asp Val Asp
   1 5 10 15
   Arg Ser Asp Ala Ser Tyr Arg Ala Trp Leu Lys Glu Ala Val Arg Lys
   20 25 30
   Val Gln Ala Asp Ala Asn Arg Ser Ala Asp Thr His Leu Leu Leu Phe
   35 40 45
   Pro Leu Pro Glu His Trp Gly Ile Asp Leu Tyr Leu Lys Asp Glu Ser
   50 55 60
   Thr His Pro Thr Gly Ser Leu Lys His Arg Leu Ala Arg Ser Leu Phe
   65 70 75 80
   Leu Tyr Gly Leu Cys Asn Gly Trp Ile Arg Pro Asp Arg Pro Val Ile
   85 90 95
   Glu Ala Ser Ser Gly Ser Thr Ala Val Ser Glu Ala Tyr Phe Ala Lys
   100 105 110
   Leu Val Gly Val Pro Phe Ile Ala Val Met Pro Arg Thr Thr Ser Ala
   115 120 125
   Glu Lys Ile Arg Leu Ile Glu Phe His Gly Gly Arg Cys His Phe Val
   130 135 140
   Asp Asp Ser Arg Arg Met Tyr Glu Glu Ser Ala Arg Leu Ala Ala Glu
   145 150 155 160
   Thr Gly Gly His Tyr Met Asp Gln Phe Thr Tyr Ala Glu Arg Ala Thr
   165 170 175
   Asp Trp Arg Gly Asn Asn Asn Ile Ala Glu Ser Ile Phe Arg Gln Leu
   180 185 190
   Arg Leu Glu Arg Tyr Pro Glu Pro Ser Trp Ile Val Ala Thr Ala Gly
   195 200 205
   Thr Gly Gly Thr Ser Ala Thr Leu Ala Arg Tyr Val His Tyr Met Gln
   210 215 220
   Tyr Asp Thr Arg Val Cys Val Ala Asp Pro Asp Asn Ser Cys Phe Phe
   225 230 235 240
   Glu Gly Trp Thr Thr Gly Asp Pro Asp Val Ala Cys Asp Arg Gly Ser
   245 250 255
   Arg Ile Glu Gly Ile Gly Arg Pro Arg Met Glu Pro Ser Phe Val Pro
   260 265 270
   Gly Ala Ile Asp Arg Met Met Lys Val Pro Asp Ala Ala Ser Val Ala
   275 280 285
   Ala Val Arg Ser Leu Glu Arg Ala Ile Gly Arg Arg Ala Gly Gly Ser
   290 295 300
   Thr Gly Thr Gly Leu Trp Ser Ala Leu Lys Ile Val Ala Glu Met Val
   305 310 315 320
   Ala Ala Gly Glu Arg Gly Ser Val Val Thr Leu Leu Cys Asp Pro Gly
   325 330 335
   Asp Arg Tyr Leu Asp Lys Tyr Tyr Ser Asp Ala Trp Leu Ala Glu Gln
   340 345 350
   Gly Leu Asp Ile Glu Pro Tyr Thr Ala Ala Ile Glu Ser Leu Leu Arg
   355 360 365
   Thr Gly Ser Leu Leu Asp
   370
<210> 51
   <211> 1104

   <212> DNA
   <213> Mycobacterium avium
<220>
   <221> CDS

   <222> (1)..(1104)
<400> 51
   gtg acg cgg acc cgg atc gcg gtc cgc aac ctg cca cgc gac tgg acg 48
   Val Thr Arg Thr Arg Ile Ala Val Arg Asn Leu Pro Arg Asp Trp Thr
   1 5 10 15
   gac aac gcg atc cgg ctg atc cag gcc gat gcg cgg cgc agc gcc gac 96
   Asp Asn Ala Ile Arg Leu Ile Gln Ala Asp Ala Arg Arg Ser Ala Asp
   20 25 30
   acc cat ctg ctg cgc tac ccg ctg ccg tcg gcc tgg gcc ggc gac gcc 144
   Thr His Leu Leu Arg Tyr Pro Leu Pro Ser Ala Trp Ala Gly Asp Ala
   35 40 45
   gac gtc gcg ctg tac ctc aag gac gag acg acg cac gtc acc ggc agc 192
   Asp Val Ala Leu Tyr Leu Lys Asp Glu Thr Thr His Val Thr Gly Ser
   50 55 60
   ctc aag cac cgg ctg gcg cgc tcg ctg ttc ctg tac gcg ctg tgc aac 240
   Leu Lys His Arg Leu Ala Arg Ser Leu Phe Leu Tyr Ala Leu Cys Asn
   65 70 75 80
   ggc tgg atc ggc gag ggc acc acg gtg gtc gag gcg tcg tcg ggg tcg 288
   Gly Trp Ile Gly Glu Gly Thr Thr Val Val Glu Ala Ser Ser Gly Ser
   85 90 95
   acg gcg gtg tcc gag gcc tac ttc gcg gcc ctg ctg ggg ctg ccg ttc 336
   Thr Ala Val Ser Glu Ala Tyr Phe Ala Ala Leu Leu Gly Leu Pro Phe
   100 105 110
   gtc gcc gtg atg ccg gag tcg acc agc gcg gcc aag gtc gcg ctg atc 384
   Val Ala Val Met Pro Glu Ser Thr Ser Ala Ala Lys Val Ala Leu Ile
   115 120 125
   gaa tca caa ggc ggc cgt tgc cat ttc gtc gcg gat tcc agc cag gtg 432
   Glu Ser Gln Gly Gly Arg Cys His Phe Val Ala Asp Ser Ser Gln Val
   130 135 140
   tac gcc gag gcc gag cgg gtg gcc cgg cag agc ggc ggg cac tac ctg 480
   Tyr Ala Glu Ala Glu Arg Val Ala Arg Gln Ser Gly Gly His Tyr Leu
   145 150 155 160
   gac cag ttc acc aac gcc gag cgg gcc acc gac tgg cgc ggc aac aac 528
   Asp Gln Phe Thr Asn Ala Glu Arg Ala Thr Asp Trp Arg Gly Asn Asn
   165 170 175
   aac atc gcc gag tcg atc ttc gag cag atg cgc gac gag aaa cac ccc 576
   Asn Ile Ala Glu Ser Ile Phe Glu Gln Met Arg Asp Glu Lys His Pro
   180 185 190
   gtg ccg gcc tgg atc gtg gtc ggc gcg ggc acc ggc ggg acg agc gcg 624
   Val Pro Ala Trp Ile Val Val Gly Ala Gly Thr Gly Gly Thr Ser Ala
   195 200 205
   acc atc ggc cgc tac atc cgc tac cgg cgg cac gcc acc cgg ctg tgc 672
   Thr Ile Gly Arg Tyr Ile Arg Tyr Arg Arg His Ala Thr Arg Leu Cys
   210 215 220
   gtc gtc gac ccg gag cac tcg gcg ttc ttc gcg gcc tac gcc gaa ggc 720
   Val Val Asp Pro Glu His Ser Ala Phe Phe Ala Ala Tyr Ala Glu Gly
   225 230 235 240
   cgg gac gac gtg gtg gcg tcc cgg tcg tcg cgg atc gag ggc atc ggc 768
   Arg Asp Asp Val Val Ala Ser Arg Ser Ser Arg Ile Glu Gly Ile Gly
   245 250 255
   cgg ccg cgg gtc gag ccg tcg ttt ctg ccc ggc gtg gtc gac cgc atg 816
   Arg Pro Arg Val Glu Pro Ser Phe Leu Pro Gly Val Val Asp Arg Met
   260 265 270
   gtc gcc gtg ccg gac gcg gcc tcc gtc gcc gcc gcc cgc cac gtc agc 864
   Val Ala Val Pro Asp Ala Ala Ser Val Ala Ala Ala Arg His Val Ser
   275 280 285
   gcc gtg ctg ggc cgg cgg gtg ggg ccc tcg acg ggc acc aac gtg tgg 912
   Ala Val Leu Gly Arg Arg Val Gly Pro Ser Thr Gly Thr Asn Val Trp
   290 295 300
   ggg gcg ttc ggc ctg ctg gcc gag ctg gtg tcg cag ggc cgc ggc ggt 960
   Gly Ala Phe Gly Leu Leu Ala Glu Leu Val Ser Gln Gly Arg Gly Gly
   305 310 315 320
   tcg gtg gtc acg ctg ctc gcc gac agc ggc gac cgg tac gcc gac acc 1008
   Ser Val Val Thr Leu Leu Ala Asp Ser Gly Asp Arg Tyr Ala Asp Thr
   325 330 335
   tac ttc gac gac gag tgg gtg gcg gcg cag ggg ctg gat ccg gcc gga 1056
   Tyr Phe Asp Asp Glu Trp Val Ala Ala Gln Gly Leu Asp Pro Ala Gly
   340 345 350
   ccg gcc gcg gcg ctg gcc gaa ttc gag cgc agt tgc gcg tgg cag tga 1104
   Pro Ala Ala Ala Leu Ala Glu Phe Glu Arg Ser Cys Ala Trp Gln
   355 360 365
<210> 52
   <211> 367
   <212> PRT
   <213> Mycobacterium avium
<400> 52
   Val Thr Arg Thr Arg Ile Ala Val Arg Asn Leu Pro Arg Asp Trp Thr
   1               5                   10                  15
   Asp Asn Ala Ile Arg Leu Ile Gln Ala Asp Ala Arg Arg Ser Ala Asp
               20                  25                  30
   Thr His Leu Leu Arg Tyr Pro Leu Pro Ser Ala Trp Ala Gly Asp Ala
           35                  40                  45
   Asp Val Ala Leu Tyr Leu Lys Asp Glu Thr Thr His Val Thr Gly Ser
       50                  55                  60
   Leu Lys His Arg Leu Ala Arg Ser Leu Phe Leu Tyr Ala Leu Cys Asn
   65                  70                  75                  80
   Gly Trp Ile Gly Glu Gly Thr Thr Val Val Glu Ala Ser Ser Gly Ser
                   85                  90                  95
   Thr Ala Val Ser Glu Ala Tyr Phe Ala Ala Leu Leu Gly Leu Pro Phe
               100                 105                 110
   Val Ala Val Met Pro Glu Ser Thr Ser Ala Ala Lys Val Ala Leu Ile
           115                 120                 125
   Glu Ser Gln Gly Gly Arg Cys His Phe Val Ala Asp Ser Ser Gln Val
       130                 135                 140
   Tyr Ala Glu Ala Glu Arg Val Ala Arg Gln Ser Gly Gly His Tyr Leu
   145                 150                 155                 160
   Asp Gln Phe Thr Asn Ala Glu Arg Ala Thr Asp Trp Arg Gly Asn Asn
                   165                 170                 175
   Asn Ile Ala Glu Ser Ile Phe Glu Gln Met Arg Asp Glu Lys His Pro
               180                 185                 190
   Val Pro Ala Trp Ile Val Val Gly Ala Gly Thr Gly Gly Thr Ser Ala
           195                 200                 205
   Thr Ile Gly Arg Tyr Ile Arg Tyr Arg Arg His Ala Thr Arg Leu Cys
       210                 215                 220
   Val Val Asp Pro Glu His Ser Ala Phe Phe Ala Ala Tyr Ala Glu Gly
   225                 230                 235                 240
   Arg Asp Asp Val Val Ala Ser Arg Ser Ser Arg Ile Glu Gly Ile Gly
                   245                 250                 255
   Arg Pro Arg Val Glu Pro Ser Phe Leu Pro Gly Val Val Asp Arg Met
               260                 265                 270
   Val Ala Val Pro Asp Ala Ala Ser Val Ala Ala Ala Arg His Val Ser
           275                 280                 285
   Ala Val Leu Gly Arg Arg Val Gly Pro Ser Thr Gly Thr Asn Val Trp
       290                 295                 300
   Gly Ala Phe Gly Leu Leu Ala Glu Leu Val Ser Gln Gly Arg Gly Gly
   305                 310                 315                 320
   Ser Val Val Thr Leu Leu Ala Asp Ser Gly Asp Arg Tyr Ala Asp Thr
                   325                 330                 335
   Tyr Phe Asp Asp Glu Trp Val Ala Ala Gln Gly Leu Asp Pro Ala Gly
               340                 345                 350
   Pro Ala Ala Ala Leu Ala Glu Phe Glu Arg Ser Cys Ala Trp Gln
           355                 360                 365
<210> 53
   <211> 40
   <212> DNA
   <213> Artificial Sequence <220>
   <223> primer for amplifying attL
<400> 53
   ctagtaagat cttgaagcct gcttttttat actaagttgg 40
<210> 54
   <211> 41
   <212> DNA
   <213> Artificial Sequence
<220>
   <223> primer for amplifying attL
<400> 54
   atgatcgaat tcgaaatcaa ataatgattt tattttgact g 41
<210> 55
   <211> 120
   <212> DNA
   <213> Escherichia coli
<400> 55
   agatcttgaa gcctgctttt ttatactaag ttggcattat aaaaaagcat tgcttatcaa   60
   tttgttgcaa cgaacaggtc actatcagtc aaaataaaat cattatttga tttcgaattc  120
<210> 56
   <211> 41
   <212> DNA
   <213> Artificial Sequence
<220>
   <223> primer for amplifying attR
<400> 56
   atgccactgc agtctgttac aggtcactaa taccatctaa g 41
<210> 57
   <211> 46
   <212> DNA
   <213> Artificial sequence
<220>
   <223> primer for amplifying attR
<400> 57
   accgttaagc tttctagacg ctcaagttag tataaaaaag ctgaac 46
<210> 58
   <211> 184
   <212> DNA
   <213> Escherichia coli
<400> 58
   ctgcagtctg ttacaggtca ctaataccat ctaagtagtt gattcatagt gactgcatat    60
   gttgtgtttt acagtattat gtagtctgtt ttttatgcaa aatctaattt aatatattga   120
   tatttatatc attttacgtt tctcgttcag cttttttata ctaacttgag cgtctagaaa   180
   gctt                                                                184
<210> 59
   <211> 38
   <212> DNA
   <213> Artificial Sequence
<220>
   <223> primer for amplifying bla
<400> 59
   ttcttagacg tcaggtggca cttttcgggg aaatgtgc 38
<210> 60
   <211> 37
   <212> DNA
   <213> Artificial Sequence
<220>
   <223> primer for amplifying bla
<400> 60
   taacagagat ctcgcgcaga aaaaaaggat ctcaaga 37
<210> 61
   <211> 46
   <212> DNA
   <213> Artificial Sequence
<220>
   <223> primer for amplifying ter_rrnB
<400> 61
   aacagagatc taagcttaga tcctttgcct ggcggcagta gcgcgg 46
<210> 62
   <211> 35
   <212> DNA
   <213> Artificial Sequence
<220>
   <223> primer for amplifying ter_rrnB
<400> 62
   ataaactgca gcaaaaagag tttgtagaaa cgcaa 35
<210> 63
   <211> 1388
   <212> DNA
   <213> Escherichia coli
<400> 63
   gaattctcat gtttgacagc ttatcatcga taagctttaa tgcggtagtt tatcacagtt     60
   aaattgctaa cgcagtcagg caccgtgtat gaaatctaac aatgcgctca tcgtcatcct    120
   cggcaccgtc accctggatg ctgtaggcat aggcttggtt atgccggtac tgccgggcct    180
   cttgcgggat atcgtccatt ccgacagcat cgccagtcac tatggcgtgc tgctagcgct    240
   atatgcgttg atgcaatttc tatgcgcacc cgttctcgga gcactgtccg accgctttgg    300
   ccgccgccca gtcctgctcg cttcgctact tggagccact atcgactacg cgatcatggc    360
   gaccacaccc gtcctgtgga tcctctacgc cggacgcatc gtggccggca tcaccggcgc    420
   cacaggtgcg gttgctggcg cctatatcgc cgacatcacc gatggggaag atcgggctcg    480
   ccacttcggg ctcatgagcg cttgtttcgg cgtgggtatg gtggcaggcc ccgtggccgg    540
   gggactgttg ggcgccatct ccttgcatgc accattcctt gcggcggcgg tgctcaacgg    600
   cctcaaccta ctactgggct gcttcctaat gcaggagtcg cataagggag agcgtcgacc    660
   gatgcccttg agagccttca acccagtcag ctccttccgg tgggcgcggg gcatgactat    720
   cgtcgccgca cttatgactg tcttctttat catgcaactc gtaggacagg tgccggcagc    780
   gctctgggtc attttcggcg aggaccgctt tcgctggagc gcgacgatga tcggcctgtc    840
   gcttgcggta ttcggaatct tgcacgccct cgctcaagcc ttcgtcactg gtcccgccac    900
   caaacgtttc ggcgagaagc aggccattat cgccggcatg gcggccgacg cgctgggcta    960
   cgtcttgctg gcgttcgcga cgcgaggctg gatggccttc cccattatga ttcttctcgc   1020
   ttccggcggc atcgggatgc ccgcgttgca ggccatgctg tccaggcagg tagatgacga   1080
   ccatcaggga cagcttcaag gatcgctcgc ggctcttacc agcctaactt cgatcactgg   1140
   accgctgatc gtcacggcga tttatgccgc ctcggcgagc acatggaacg ggttggcatg   1200
   gattgtaggc gccgccctat accttgtctg cctccccgcg ttgcgtcgcg gtgcatggag   1260
   ccgggccacc tcgacctgaa tggaagccgg cggcacctcg ctaacggatt caccactcca   1320
   actagaaagc ttaacacaga aaaaagcccg cacctgacag tgcgggcttt ttttttcgac   1380
   cactgcag                                                            1388
<210> 64
   <211> 36
   <212> DNA
   <213> Artificial Sequence
<220>
   <223> primer for amplifying ter_thrL
<400> 64
   agtaattcta gaaagcttaa cacagaaaaa agcccg 36
<210> 65
   <211> 43
   <212> DNA
   <213> Artificial Sequence
<220>
   <223> primer for amplifying ter_thrL
<400> 65
   ctagtaggat ccctgcagtg gtcgaaaaaa aaagcccgca ctg 43
<210> 66
   <211> 501
   <212> DNA
   <213> Pantoea ananatis
<220>
   <221> CDS
   <222> (1)..(501)
<400> 66
   atg gcg gta atg gag aaa ata ttt cta ccg gag ggc cgt atg cca gat      48
   Met Ala Val Met Glu Lys Ile Phe Leu Pro Glu Gly Arg Met Pro Asp
   1               5                   10                  15
   aaa att gac ctt aaa ctg ttg gct atg ctg cag cag gac tgc acc act      96
   Lys Ile Asp Leu Lys Leu Leu Ala Met Leu Gln Gln Asp Cys Thr Thr
               20                  25                  30
   tca ctc cag gta ctg gca gat gcg gtt aat ctc acc acg acg ccc tgt     144
   Ser Leu Gln Val Leu Ala Asp Ala Val Asn Leu Thr Thr Thr Pro Cys
           35                  40                  45
   tgg aag cgc ctt aaa aag ctg gaa gaa gac ggc att att cgc gga cgg     192
   Trp Lys Arg Leu Lys Lys Leu Glu Glu Asp Gly Ile Ile Arg Gly Arg
       50                  55                  60
   gtc gct ctg ctg gat aat gaa aag ctt ggc ctc tgt ctc acg gcc ttt     240
   Val Ala Leu Leu Asp Asn Glu Lys Leu Gly Leu Cys Leu Thr Ala Phe
   65                  70                  75                  80
   atg ttt gtc aaa acc acc cag cac agc aaa gcc tgg tat cag gag ttc     288
   Met Phe Val Lys Thr Thr Gln His Ser Lys Ala Trp Tyr Gln Glu Phe
                   85                  90                  95
   gtc tcg gtg gtg cag agc atg ccg gaa gtg atg ggc ttt tac cgt atg     336
   Val Ser Val Val Gln Ser Met Pro Glu Val Met Gly Phe Tyr Arg Met
               100                 105                 110
   gcc ggt gag tac gat tat tta ctg cgt att cag gtt gcc gac atg aaa     384
   Ala Gly Glu Tyr Asp Tyr Leu Leu Arg Ile Gln Val Ala Asp Met Lys
           115                 120                 125
   agt tat gat gcc ttt tat aag cgt tta gtt aat ggt gta aca ggc ctg     432
   Ser Tyr Asp Ala Phe Tyr Lys Arg Leu Val Asn Gly Val Thr Gly Leu
       130                 135                 140
   atc gat gtg acc tcc agc ttc gcg atg gaa gag att aaa tac acg aca     480
   Ile Asp Val Thr Ser Ser Phe Ala Met Glu Glu Ile Lys Tyr Thr Thr
   145                 150                 155                 160
   gcc ctg cct gtt ggc ccc tga                                         501
   Ala Leu Pro Val Gly Pro
                   165
<210> 67
   <211> 166
   <212> PRT
   <213> Pantoea ananatis
<400> 67
   Met Ala Val Met Glu Lys Ile Phe Leu Pro Glu Gly Arg Met Pro Asp
   1               5                   10                  15
   Lys Ile Asp Leu Lys Leu Leu Ala Met Leu Gln Gln Asp Cys Thr Thr
               20                  25                  30
   Ser Leu Gln Val Leu Ala Asp Ala Val Asn Leu Thr Thr Thr Pro Cys
           35                  40                  45
   Trp Lys Arg Leu Lys Lys Leu Glu Glu Asp Gly Ile Ile Arg Gly Arg
       50                  55                  60
   Val Ala Leu Leu Asp Asn Glu Lys Leu Gly Leu Cys Leu Thr Ala Phe
   65                  70                  75                  80
   Met Phe Val Lys Thr Thr Gln His Ser Lys Ala Trp Tyr Gln Glu Phe
                   85                  90                  95
   Val Ser Val Val Gln Ser Met Pro Glu Val Met Gly Phe Tyr Arg Met
               100                 105                 110
   Ala Gly Glu Tyr Asp Tyr Leu Leu Arg Ile Gln Val Ala Asp Met Lys
           115                 120                 125
   Ser Tyr Asp Ala Phe Tyr Lys Arg Leu Val Asn Gly Val Thr Gly Leu
       130                 135                 140
   Ile Asp Val Thr Ser Ser Phe Ala Met Glu Glu Ile Lys Tyr Thr Thr
   145                 150                 155                 160
   Ala Leu Pro Val Gly Pro
                   165